# EUROPEAN PATENT APPLICATION

(11) **EP 3 329 923 A1**
(43) Date of publication of application: **06.06.2018**
(21) Application number: 16202005.1
(22) Date of filing: 02.12.2016
(51) Int. Cl.: A61K 31/7068, A61K 31/5355, A61K 45/06, A61P 35/00

(54) **THE POTENTIAL OF CRBN-INDEPENDENT IMID RESENSITIZATION BY EPIGENETIC THERAPY**

(71) Applicant: University of Copenhagen, 1165 Copenhagen K (DK); Rigshospitalet, 2100 Copenhagen (DK)
(72) Inventor: Dimopoulos, Konstantinos, 2100 Copenhagen (DK); Grønbæk, Kirsten, 2100 Copenhagen (DK)
(74) Representative: Aera A/S

(57) **Abstract**

The present invention relates to an epigenetic treatment with a combination of 5-Aza and EPZ-6438 for reversing chromatin accessibility and re-establishment of IMiD sensitivity in cancers such at but not limited to Multiple Myeloma.

## Description

### FIELD OF THE INVENTION

This disclosure relates to a combination of inhibitors of human histone methyltransferase EZH2 and Azacitidine, and methods of combination therapy for treating cancer, especially Multiple myeloma by ImiD resensitization.

### BACKGROUND OF THE INVENTION

Multiple myeloma (MM) is a devastating, malignant disease, often characterized by severe bone pain and renal failure.

Therapeutic advancement in recent years has increased the overall survival of MM patients. This is due not only to autologous Stem Cell Transplantation (SCT), but also to the availability of novel agents, such as the Immunomodulatory Drugs (IMiDs) thalidomide, lenalidomide, and pomalidomide.

Despite the use of these IMiD agents, MM relapses in virtually all patients.

The development of resistance to therapy is unavoidable in the history of multiple myeloma patients. Therefore, there is an unmet need for the study of the its characteristics and mechanisms of MM, which is critical in the search for novel therapeutic approaches to overcome it.

Despite the fact that novel drugs such as lenalidomide and pomalidomide have improved the quality of life and survival rate of patients with MM, the disease is still considered incurable.

The precise mechanism of action of these drugs was recently suggested to involve a protein called cereblon (CRBN), as their biological target. Low expression of CRBN is associated with resistance to these drugs, but it is still not known how the expression of CRBN is regulated in patients with MM.

In addition, it is still not known whether the development of IMiD resistance exclusively depends on CRBN downregulation, or whether there are additional mechanisms.

### SUMMARY OF THE INVENTION

The present inventors found that epigenetic changes such as chromatin accessibility was profoundly changed in the resistant cell lines disclosed herein, but restored after treatment with the inventive epidrugs of the present invention. Thus, the present invention relates to the use of an EZH2 inhibitor and a cytidine analogue in the treatment of cancer by epigenetic resensitization.

In a first aspect, the present invention relates to a combination of an EZH2 inhibitor and a cytidine analogue for use in the treatment of cancer, and in particular to scenarios wherein the cancer is resistant to Immunomodulatory drugs (IMiDs) chemotherapy.

Another aspect of the present invention relates to a composition for use in treating a cancer, the composition comprises a first component consisting of an effective amount of an EZH2 inhibitor and a second component comprising an effective amount of a cytidine analogue. Such compositions are useful for securing that epigenetic changes in the cancer cells are restored to states where normal chemotherapy becomes more effective.

A third aspect of the present invention relates to an effective amount of an EZH2 inhibitor and a cytidine analogue for use in the preparation of a medicament for treating cancer. Such a medicament is for example capable of changing the chromatin accessibility.

In a fourth aspect, the EZH2 inhibitor and a cytidine analogue can be combined with an Immunomodulatory drug (IMiD), preferably Lenalidomide and/or Pomalidomide.

One molecular aspect of the present invention is the capability of reversing drug resistance in a patient having an Immunomodulatory drug (IMiD) resistant cancer comprising administering to said patient a combination of an EZH2 inhibitor and a cytidine analogue.

Another aspect of the present invention relates to a method for treating cancer comprising administering to a subject in need thereof
(a) an effective amount of an EZH2 inhibitor, and
(b) an effective amount of a cytidine analogue
to provide a combination therapy having an enhanced therapeutic effect compared to the effect of the EZH2 inhibitor and the cytidine analogue administered alone. This combination therapy can provide a synergistic therapeutic effect.

Finally, the present invention also relates to kits for treating cancer at least comprising
(a) an effective amount of an EZH2 inhibitor and
(b) an effective amount of a cytidine analogue, and
(c) optionally instruction for use thereof.

### DETAILED DESCRIPTION OF THE INVENTION

The present inventors have identified the epigenetic mechanisms that are responsible for downregulation of CRBN and resistance to IMiDs in cancers such as Multiple Myeloma (MM).

The present inventors have elucidated the mechanisms of development of IMiD resistance, and identified molecular biomarkers that can predict the clinical response to IMiDs.

Since the mechanisms that contribute to IMiD resistance are reversible, the present inventors are able to develop drugs that can restore the sensitivity of the malignant cells to IMiDs, which will improve the quality of life and survival rate of patients with MM, a disease that is presently considered incurable. These drugs are a combination of an EZH2 inhibitor and a cytidine analogue, the latter also known as DNMTi.

Thus, one embodiment of the present invention relates to a combination of an EZH2 inhibitor and a cytidine analogue for use in the treatment in cancer, preferably MM.

### The combination of an EZH2 inhibitor and a cytidine analogue

In the present context, then one benefit by the combination of an EZH2 inhibitor and a cytidine analogue during therapy is - that with one drug alone, a cell that acquires a mutation that confers resistance to the drug will be at a proliferative advantage. By the time this is recognized and treatment with a new or second drug is started, it is likely that a cell resistant to both drugs will already have emerged.

Starting therapy with both drugs simultaneously or within a close time frame means that cells acquiring single resistance will be immediately eliminated by the other drug. This approach increases the chance of effective treatment. In the present context, the combination of the two drugs functions as a means for reversing drug resistance rather than as a traditional chemotherapeutic drug. Thus, in certain aspects, such "combination therapy" also embraces the administration of the therapeutic agents as described above in further combination with other biologically active ingredients and non-drug therapies (e.g., surgery or radiation treatment).

Where the combination therapy further comprises a non-drug treatment, the non-drug treatment may be conducted at any suitable time so long as a beneficial effect from the co-action of the combination of the therapeutic agents and non-drug treatment is achieved.

For example, in appropriate cases, the beneficial effect is still achieved when the non-drug treatment is temporally removed from the administration of the therapeutic agents, perhaps by days or even weeks.

### EZH2 inhibitor

An EZH2 inhibitor in the present context is an inhibitor of EZH2, a histone methyl transferase subunit of a Polycomb Repressive Complex 2 (PRC2), which is recurrently mutated in several forms of cancer and is highly expressed in numerous others.

EZH2 inhibits genes responsible for suppressing tumor development, and blocking EZH2 activity may slow tumor growth. EZH2 has been targeted for inhibition because it is upregulated in multiple cancers including, but not limited to, breast, prostate, melanoma, and bladder cancer.

Mutation or over-expression of EZH2 has been linked to many forms of cancer.

Developing an inhibitor of EZH2 and preventing unwanted histone methylation of tumor suppressor genes is a viable area of cancer research. EZH2 inhibitor development has focused on targeting the SET domain active site of the protein.

Several inhibitors of EZH2 have been developed such as but not limited to 3-deazaneplanocin A (DZNep), EPZ005687, tazemetostat (EPZ-6438), Sinefungin, EI1, GSK126, and UNC1999.

### 3-deazaneplanocin A (DZNep)

In one embodiment, the EZH2 inhibitor of the present invention is a DZNep.

3-deazaneplanocin A (DZNep) has potential antiviral and anti-cancer properties because it lowers EZH2 levels and induces apoptosis in breast and colon cancer cells. DZNep inhibits the demethylation of S-adenosyl-L-methionine, the cofactor of EZH2, to form S-adenosyl-L-homocysteine, therefore blocking the transfer of the methyl group to a histone. However, DZNep is not specific to EZH2, and also inhibits other DNA methyltransferases.

### EPZ005687, an S-adenosylmethionine (SAM) inhibitor

In one embodiment, the EZH2 inhibitor of the present invention is EPZ005687.

EPZ005687 is an S-adenosylmethionine (SAM) inhibitor that is more selective than DZNep; it has a 50-fold increase in selectivity for EZH2 compared to EZH1. The drug blocks EZH2 activity by binding to the SET domain active site of the enzyme. EPZ005687 can also inhibit the Y641 and A677 mutants of EZH2, which may be applicable for treating non-Hodgkin's lymphoma.

### Tazemetostat (EPZ-6438)

In one embodiment, the EZH2 inhibitor of the present invention is Tazemetostat (EPZ-6438), which has the following chemical structure N-((4,6-dimethyl-2-oxo-1,2-dihydropyridin-3-yl)methyl)-5-(ethyl(tetrahydro-2H-pyran-4-yl)amino)-4-methyl-4'-(morpholinomethyl)-[1,1'-biphenyl]-3-carboxamide.

Tazemetostat is an orally available, small molecule selective and S-adenosyl methionine (SAM) competitive inhibitor of histone methyl transferase EZH2, with potential antineoplastic activity, also known as CAS 1403254-99-8, E7438, EPZ6438, and EPZ-6438 - these terms are re used interchangeably herein.

Upon oral administration, EPZ-6438 selectively inhibits the activity of both wild-type and mutated forms of EZH2. Inhibition of EZH2 specifically prevents the methylation of histone H3 lysine 27 (H3K27).

This decrease in histone methylation alters gene expression patterns associated with cancer pathways and results in decreased tumor cell proliferation in EZH2 mutated cancer cells. EZH2, which belongs to the class of histone methyltransferases (HMTs), is overexpressed or mutated in a variety of cancer cells and plays a key role in tumor cell proliferation.

EPZ-6438 or a pharmaceutically acceptable salt thereof, as described herein, is potent in targeting both WT and mutant EZH2. EPZ-6438 is orally bioavailable and has high selectivity to EZH2 compared with other histone methyltransferases (i.e. >20,000 fold selectivity by Ki). Importantly, EPZ-6438 has target methyl mark inhibition that results in the killing of genetically defined cancer cells in vitro. Animal models have also shown sustained in vivo efficacy following inhibition of target methyl mark. Clinical trial results described herein also demonstrate the safety and efficacy of EPZ-6438.

### Sinefungin

In one embodiment, the EZH2 inhibitor of the present invention is Sinefungin.

Sinefungin is another SAM-competitive inhibitor similar to DZNep, however, like DZNep, it is not specific to EZH2. It works by binding in the cofactor binding pocket of DNA methyltransferases to block methyl transfer.

### EI1

In one embodiment, the EZH2 inhibitor of the present invention is EI1.

EI1 is another inhibitor that showed EZH2 inhibitory activity in lymphoma tumor cells, including cells with the Y641 mutation. The mechanism of this inhibitor also involves competing with the SAM cofactor for binding to EZH2.

### GSK126

In one embodiment, the EZH2 inhibitor of the present invention is GSK126.

GSK126 is a potent, SAM-competitive EZH2 inhibitor, that has 150-fold selectivity over EZH1 and a Ki of 0.5-3 nM.

### UNC1999

In one embodiment, the EZH2 inhibitor of the present invention is UNC1999.

UNC1999 was developed as an analogue of GSK126, and was the first orally bioavailable EZH2 inhibitor to show activity. However, it is less selective than its counterpart GSK126, and it binds to EZH1 as well, increasing the potential for off-target effects.

### Cytidine analogues - DNA methyltransferase inhibitors (DNMTi)

A cytidine analogue in the present context relates to any chemical analogue of cytidine, a nucleoside in DNA and RNA. There are a variety of cytidine analogues for example, KP-1461, zebularine, azacitidine and its analog decitabine.

### KP-1461

In one embodiment, the cytidine analogue of the present invention is carbamic acid, (5-(2-deoxy-beta-D-erythro-pentofuranosyl)-1,4,5,6-tetrahydro-4-oxo-1,3,5-triazin-2-yl)-, heptyl ester commercially known as KP-1461.

KP-1461 is a prodrug. In the body, KP-1461 is first converted to KP-1212. Then, once KP-1212 is inside cells, it is converted to an active form called KP-1212-TP.

### Zebularine

In one embodiment, the cytidine analogue of the present invention is 1-(β-D-Ribofuranosyl)-2(1H)-pyrimidinone commercially know as Zebularine.

Zebularine inhibits DNA methylation and tumor growth both in vitro and in vivo.

### Azacitidine

Azacitidine is a pyrimidine nucleoside analogue of cytidine with antineoplastic activity having the chemical structure 4-amino-1-beta-D-ribofuranosyl-1,3,5-triazin-2(1H)-one.

Azacitidine is incorporated into DNA, where it reversibly inhibits DNA methyltransferase, thereby blocking DNA methylation. Hypomethylation of DNA by azacitidine may activate tumor suppressor genes silenced by hypermethylation, resulting in an antitumor effect. This agent is also incorporated into RNA, thereby disrupting normal RNA function and impairing tRNA cytosine-5-methyltransferase activity.

Azacitidine is also known as 5-AC, 5-azacytidine, azacytidine or ladakamycin or under the trade name Vidaza or Mylosar and these terms are used interchangeably herein.

In one embodiment, the cytidine analogue of the present invention is Azacitidine and its deoxy derivative, decitabine (also known as 5-aza-2'deoxycytidine).

### Decitabine

Decitabine having the chemical structure 4-Amino-1-(2-deoxy-β-D-erythro-pentofuranosyl)-1,3,5-triazin-2(1H)-one has shown results against cancer through epigenetic demethylation.

### Specific combination

One embodiment of the present invention relates to a combination of an EZH2 inhibitor and a cytidine analogue for use in the treatment of cancer, wherein the EZH2 inhibitor is EPZ-6438 and a cytidine analogue is Azacitidine.

One embodiment of the present invention relates to a combination of an EZH2 inhibitor and a cytidine analogue for use in the treatment of cancer, wherein the EZH2 inhibitor is DZNep and a cytidine analogue is Azacitidine.

One embodiment of the present invention relates to a combination of an EZH2 inhibitor and a cytidine analogue for use in the treatment of cancer, wherein the EZH2 inhibitor is EPZ-005687 and a cytidine analogue is Azacitidine.

One embodiment of the present invention relates to a combination of an EZH2 inhibitor and a cytidine analogue for use in the treatment of cancer, wherein the EZH2 inhibitor is Sinefungin and a cytidine analogue is Azacitidine.

One embodiment of the present invention relates to a combination of an EZH2 inhibitor and a cytidine analogue for use in the treatment of cancer, wherein the EZH2 inhibitor is EI1 and a cytidine analogue is Azacitidine.

One embodiment of the present invention relates to a combination of an EZH2 inhibitor and a cytidine analogue for use in the treatment of cancer, wherein the EZH2 inhibitor is GSK126 and a cytidine analogue is Azacitidine.

One embodiment of the present invention relates to a combination of an EZH2 inhibitor and a cytidine analogue for use in the treatment of cancer, wherein the EZH2 inhibitor is UNC1999 and a cytidine analogue is Azacitidine.

One embodiment of the present invention relates to a combination of an EZH2 inhibitor and a cytidine analogue for use in the treatment of cancer, wherein the EZH2 inhibitor is EPZ-6438 and a cytidine analogue is KP-1461.

One embodiment of the present invention relates to a combination of an EZH2 inhibitor and a cytidine analogue for use in the treatment of cancer, wherein the EZH2 inhibitor is DZNep and a cytidine analogue is KP-1461.

One embodiment of the present invention relates to a combination of an EZH2 inhibitor and a cytidine analogue for use in the treatment of cancer, wherein the EZH2 inhibitor is EPZ-005687 and a cytidine analogue is KP-1461.

One embodiment of the present invention relates to a combination of an EZH2 inhibitor and a cytidine analogue for use in the treatment of cancer, wherein the EZH2 inhibitor is Sinefungin and a cytidine analogue is KP-1461.

One embodiment of the present invention relates to a combination of an EZH2 inhibitor and a cytidine analogue for use in the treatment of cancer, wherein the EZH2 inhibitor is EI1 and a cytidine analogue is KP-1461.

One embodiment of the present invention relates to a combination of an EZH2 inhibitor and a cytidine analogue for use in the treatment of cancer, wherein the EZH2 inhibitor is GSK126 and a cytidine analogue is KP-1461.

One embodiment of the present invention relates to a combination of an EZH2 inhibitor and a cytidine analogue for use in the treatment of cancer, wherein the EZH2 inhibitor is UNC1999 and a cytidine analogue is KP-1461.

One embodiment of the present invention relates to a combination of an EZH2 inhibitor and a cytidine analogue for use in the treatment of cancer, wherein the EZH2 inhibitor is EPZ-6438 and a cytidine analogue is Zebularine.

One embodiment of the present invention relates to a combination of an EZH2 inhibitor and a cytidine analogue for use in the treatment of cancer, wherein the EZH2 inhibitor is DZNep and a cytidine analogue is Zebularine.

One embodiment of the present invention relates to a combination of an EZH2 inhibitor and a cytidine analogue for use in the treatment of cancer, wherein the EZH2 inhibitor is EPZ-005687 and a cytidine analogue is Zebularine.

One embodiment of the present invention relates to a combination of an EZH2 inhibitor and a cytidine analogue for use in the treatment of cancer, wherein the EZH2 inhibitor is Sinefungin and a cytidine analogue is Zebularine.

One embodiment of the present invention relates to a combination of an EZH2 inhibitor and a cytidine analogue for use in the treatment of cancer, wherein the EZH2 inhibitor is EI1 and a cytidine analogue is Zebularine.

One embodiment of the present invention relates to a combination of an EZH2 inhibitor and a cytidine analogue for use in the treatment of cancer, wherein the EZH2 inhibitor is GSK126 and a cytidine analogue is Zebularine.

One embodiment of the present invention relates to a combination of an EZH2 inhibitor and a cytidine analogue for use in the treatment of cancer, wherein the EZH2 inhibitor is UNC1999 and a cytidine analogue is Zebularine.

One embodiment of the present invention relates to a combination of an EZH2 inhibitor and a cytidine analogue for use in the treatment of cancer, wherein the EZH2 inhibitor is EPZ-6438 and a cytidine analogue is Decitabine.

One embodiment of the present invention relates to a combination of an EZH2 inhibitor and a cytidine analogue for use in the treatment of cancer, wherein the EZH2 inhibitor is DZNep and a cytidine analogue is Decitabine.

One embodiment of the present invention relates to a combination of an EZH2 inhibitor and a cytidine analogue for use in the treatment of cancer, wherein the EZH2 inhibitor is EPZ-005687 and a cytidine analogue is Decitabine.

One embodiment of the present invention relates to a combination of an EZH2 inhibitor and a cytidine analogue for use in the treatment of cancer, wherein the EZH2 inhibitor is Sinefungin and a cytidine analogue is Decitabine.

One embodiment of the present invention relates to a combination of an EZH2 inhibitor and a cytidine analogue for use in the treatment of cancer, wherein the EZH2 inhibitor is EI1 and a cytidine analogue is Decitabine.

One embodiment of the present invention relates to a combination of an EZH2 inhibitor and a cytidine analogue for use in the treatment of cancer, wherein the EZH2 inhibitor is GSK126 and a cytidine analogue is Decitabine.

One embodiment of the present invention relates to a combination of an EZH2 inhibitor and a cytidine analogue for use in the treatment of cancer, wherein the EZH2 inhibitor is UNC1999 and a cytidine analogue is Decitabine.

### Immunomodulatory drugs (IMiDS)

Thalidomide and its derivatives lenalidomide and pomalidomide represent a new class of antineoplastic compounds called IMiDs, which have immune-modulatory, anti-in ammatory, and anti-angiogenic properties. Their mechanism of action was elucidated only recently, after the discovery of their receptor, the Cereblon protein (CRBN). IMiDs are able to modify the substrate specificity of CRBN, and induce the proteasome degradation of the ikaros proteins IKZF1 and IKZF3. In view of these discoveries, the IMiDs should be more properly called "ubiquitin ligase modulators".

The present inventors have developed lenalidomide- and pomalidomide-resistant counterparts of both OPM2 and NCI-H929 (OPM2LR and OPM2PR, NLI-H929-LR and NCI-H929PR, respectively), by continuously culturing with IMiDs until they also lost their sensitivity to the drugs.

### Lenalidomide

Lenalidomide is a second-generation IMiD, that was developed from the structural backbone of the thalidomide molecule by the addition of an amino group (NH2-) at position 4 of the phthaloyl ring and removal of the carbonyl group (C = O) of the 4-amino-substituted phthaloyl ring.

### Pomalidomide

Pomalidomide (CC4047) is another novel compound shown to have promising activity in MM and myelofibrosis. It was developed by modifying thalidomide structure via the addition of an amino acid at the position 4 of the phthaloyl ring. In vitro studies showed that the 4-amino analogs were up to 50,000-times more potent inhibitors of TNF-α than the parent compound.

### Resistance to Immunomodulatory drugs (IMiDs) chemotherapy

In an effort to understand whether resistance to IMiDs is reversible, the present inventors have treated all the resistant cell lines with diverse epigenetic drugs such as but not limited to 5-Azacytidine, EPZ-6438, curcumin and Panabinostat both alone and in combination.

The data presented herein showed that in particular the combination of 5-Azacytidinne and EPZ-6438 could very effectively restore sensitivity to IMiDs in all the cells with acquired resistance. Resistance to IMiDs have been associated with e.g. induction of the Wnt/β-catenin pathway and decreased expression of the CRBN protein.

Thus, in one embodiment the present invention relates to combination therapies when primary treatments begin to fail because the cancer is resistant to Immunomodulatory drugs (IMiDs) chemotherapy. The present invention relates to an EZH2 inhibitor and a Cytidine analogues for use in reversing the resistance to IMiD chemotherapy-resistant cancers, and thereby provide a treatment of IMiD chemotherapy-resistant cancers, such as MM.

The molecular mechanisms supporting the present invention is disclosed in the EXAMPLES, and in short they are that
The CRBN downregulation in acquired IMiD resistance is not due to promoter DNA methylation or histone 3 lysine 27 trimethylation
The acquired resistance to IMiDs is associated with more chromatine accesibility changes than DNA methylation changes, and
Epigenetic treatment with combination of 5-Aza and EPZ-6438 can reverse chromatin accessibility and reestablish IMiD sensitivity in cell lines with acquired resistance, an event which is CRBN-independent

Furthermore, as also disclosed in the EXAMPLES, then the present inventors show indications that EPZ-6438 can potentially sensitize myeloma cells with intrinsic resistance to IMiDs. Thus, in one embodiment, the present invention relates to an EZH2 inhibitor for use in reversing the resistance to IMiD chemotherapy-resistant cancers, and thereby provide a treatment of IMiD chemotherapy-resistant cancers, such as MM.

In one embodiment, the present invention relates to an EPZ-6438 inhibitor for use in reversing the resistance to IMiD chemotherapy-resistant cancers, and thereby provide a treatment of IMiD chemotherapy-resistant cancers, such as MM.

In one embodiment, the present invention relates to an DZNep inhibitor for use in reversing the resistance to IMiD chemotherapy-resistant cancers, and thereby provide a treatment of IMiD chemotherapy-resistant cancers, such as MM.

In one embodiment, the present invention relates to an EPZ005687 inhibitor for use in reversing the resistance to IMiD chemotherapy-resistant cancers, and thereby provide a treatment of IMiD chemotherapy-resistant cancers, such as MM.

In one embodiment, the present invention relates to a Sinefungin inhibitor for use in reversing the resistance to IMiD chemotherapy-resistant cancers, and thereby provide a treatment of IMiD chemotherapy-resistant cancers, such as MM.

In one embodiment, the present invention relates to an EI1 inhibitor for use in reversing the resistance to IMiD chemotherapy-resistant cancers, and thereby provide a treatment of IMiD chemotherapy-resistant cancers, such as MM.

In one embodiment, the present invention relates to a GSK126 inhibitor for use in reversing the resistance to IMiD chemotherapy-resistant cancers, and thereby provide a treatment of IMiD chemotherapy-resistant cancers, such as MM.

In one embodiment, the present invention relates to an UNC1999 inhibitor for use in reversing the resistance to IMiD chemotherapy-resistant cancers, and thereby provide a treatment of IMiD chemotherapy-resistant cancers, such as MM.

### Epigenetic changes

Cereblon (CRBN) is the primary target of thalidomide teratogenicity. Thalidomide binds to CRBN, alters the function of the E3 ubiquitin ligase complex, and induces downstream effects, including cell cycle arrest caused by the upregulation of the cyclin-dependent kinase inhibitor and the downregulation of interferon regulatory factor 4 (IRF4), which targets critical genes, including MYC, CDK6, and CASP.

CRBN is also required for the anti-MM action of the thalidomide derivatives lenalidomide and pomalidomide; thus decreasing the expression of CRBN results in resistance to IMiDs, as evidenced by both in vitro and clinical studies.

In an effort to understand the regulation of CRBN and elucidate the mechanisms of IMiD resistance, the present inventors focused on epigenetic changes, such as DNA methylation, histone marks and nucleosome positioning (chromatin accessibility).

Even though none of the aforementioned mechanisms was found to be responsible for the regulation of CRBN (see examples 4, 5, 7), the present inventors discovered that acquired resistance to IMiDs was associated with epigenetic changes, primarily a decrease in chromatin accessibility (see example 5)

Thus, there is a need for restoring these epigenetic changes in cancer cells having acquired resistances to IMiDs, such as Thalidomide and its derivatives lenalidomide and pomalidomide.

In one embodiment, the invention relates to resensitization of IMiD resistance by altering the epigenetics of the cancer cells with a combination of an EZH2 inhibitor such as but not limited to Tazemetostat, and a Cytidine analogue such as but not limited to Azactidine.

In one embodiment, the alteration of the epigenetics of the cancer cells may be selected from the group consisting of DNA methylation, histone marks and nucleosome positioning (chromatin accessibility).

In one embodiment, the alteration of the epigenetics is nucleosome positioning (chromatin accessibility).

In one embodiment, the alteration of the epigenetics is DNA methylation.

In one embodiment, the alteration of the epigenetics is histone marks.

In one embodiment, the alteration of the epigenetics is CRBN independent.

### Resensitization - reverse the resistance

There are plenty of mechanisms that contribute to drug resistance of cancer cells, with the most common being gene mutations and epigenetic changes. In contrary with gene mutations, which cannot be reversed, epigenetic changes have been shown to be potentially reversible (for example DNA methylation, by using DNA-methyltransferase inhibitors).

Since resistance to IMiDs was shown to be associated with epigenetic changes (primarily chromatin accessibility), the present inventors examined whether sensitivity to IMiDs could be restored by treating the resistant cells with epigenetic drugs (5-Azacytidine, Panobinostat, EPZ-6438, curcumin).

In particular, the combination of 5-Azacytidine and EPZ-6438 was shown to be extremely effective in resensitizing the IMiD-resistant cells to both lenalidomide and pomalidomide (see example 6).

Thus, one embodiment of the present invention relates to the use of a combination of an EZH2 inhibitor and a cytidine analogue for resensitization of cancer cells to IMiDs, wherein the epigenetic changes that initially lead to the resistance in the cancer cells are restored.

Interestingly, the process of resensitization did not involve CRBN. Firstly, even though CRBN was downregulated in acquired IMiD resistance (see example 2), it was not found to be regulated by any epigenetic changes. Furthermore, the expression of CRBN remained unchanged, despite the effective resensitization of resistant cells to IMiDs through treatment with 5-Azacytidine and EPZ-6438 (see example 7)

In one embodiment, the epigenetic changes that resensitize the cancer cells to IMiDs are CRBN independent.

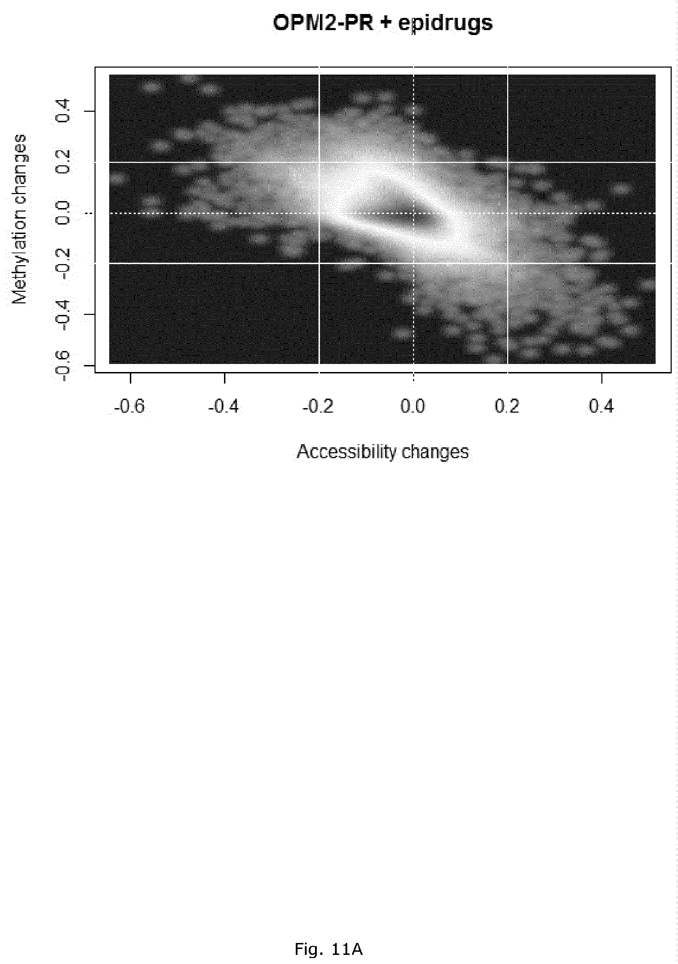

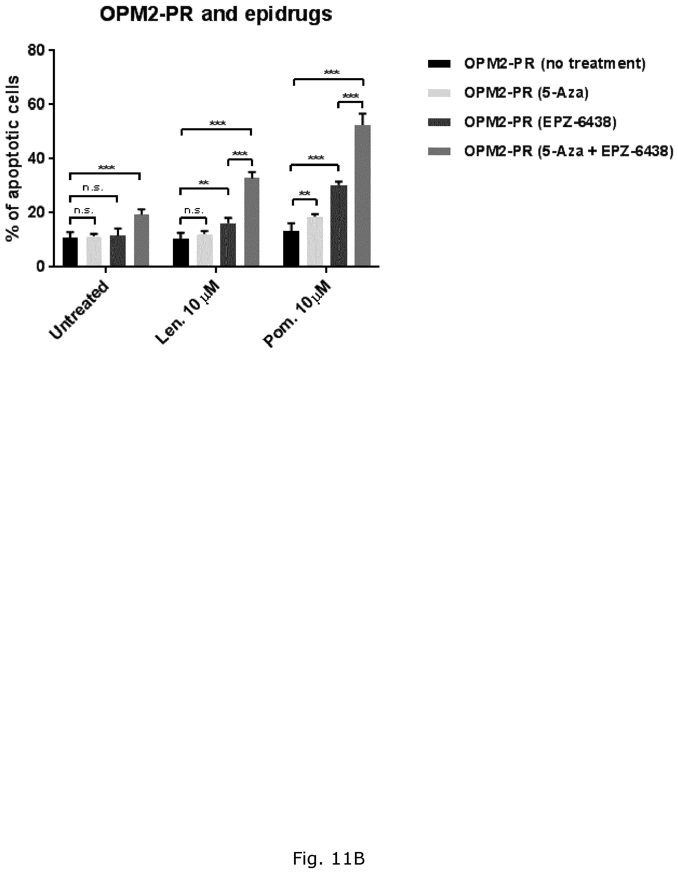

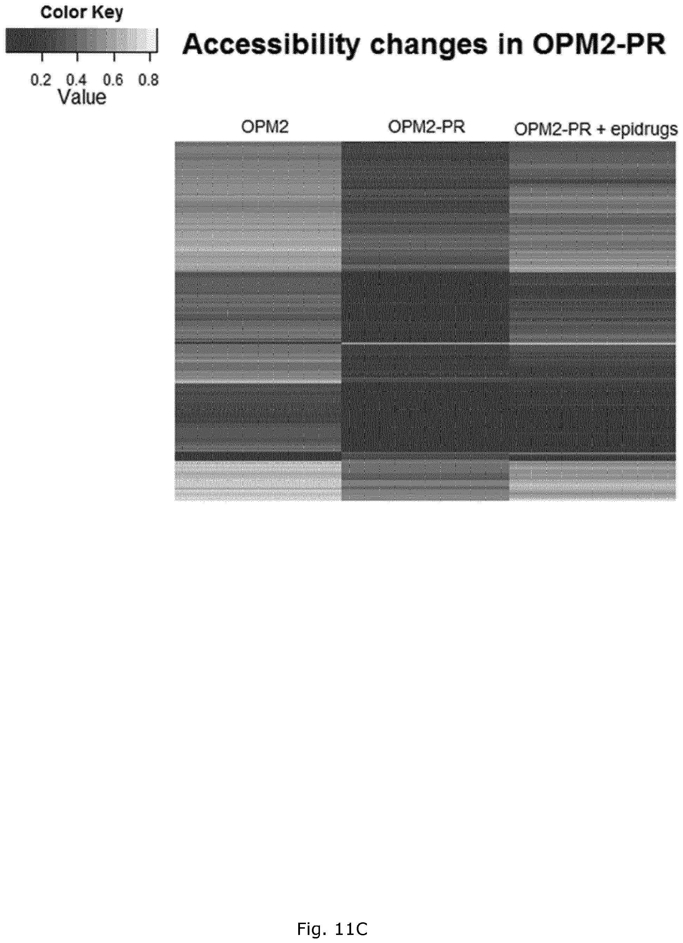

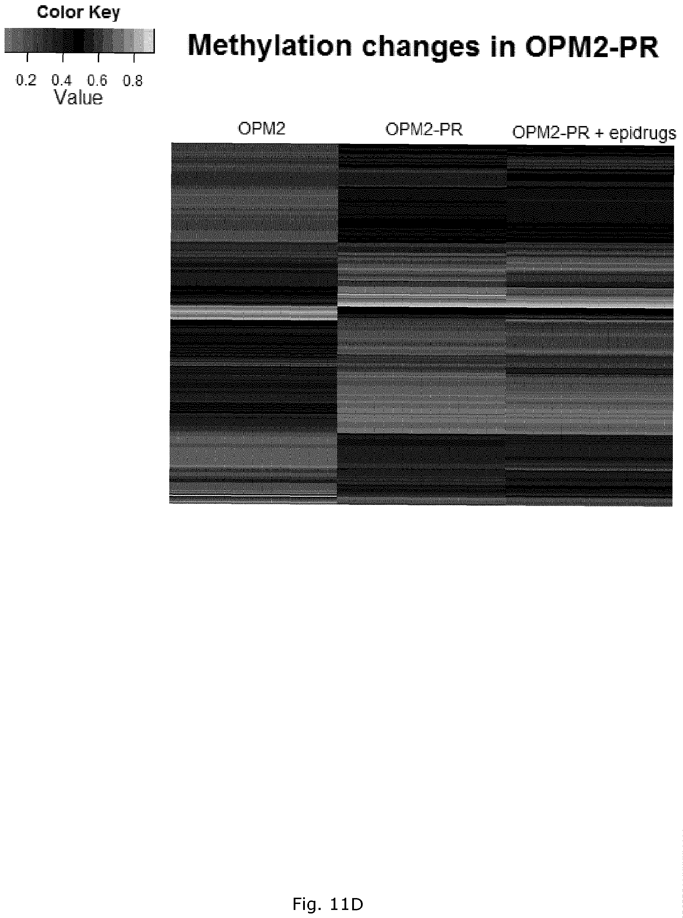

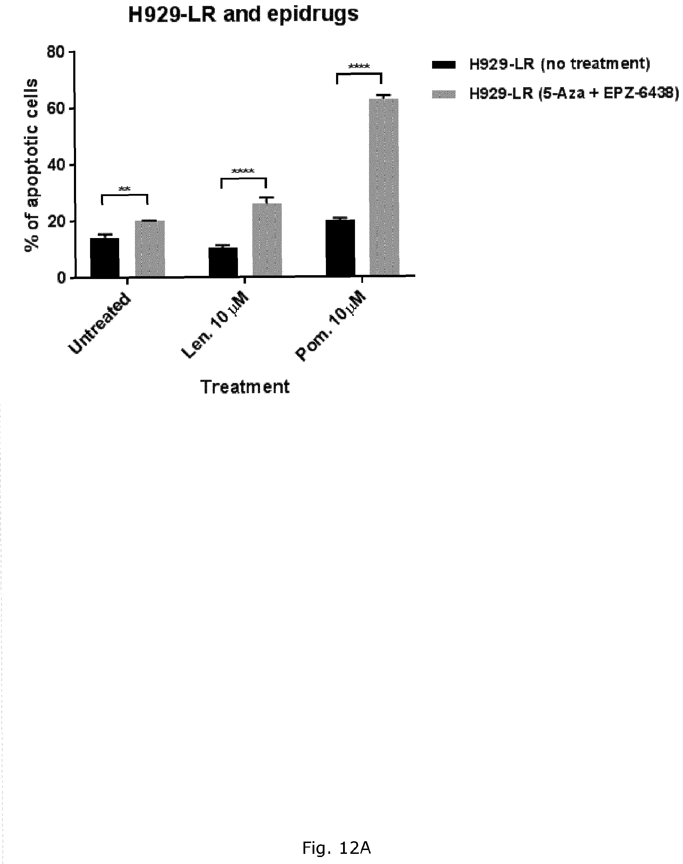

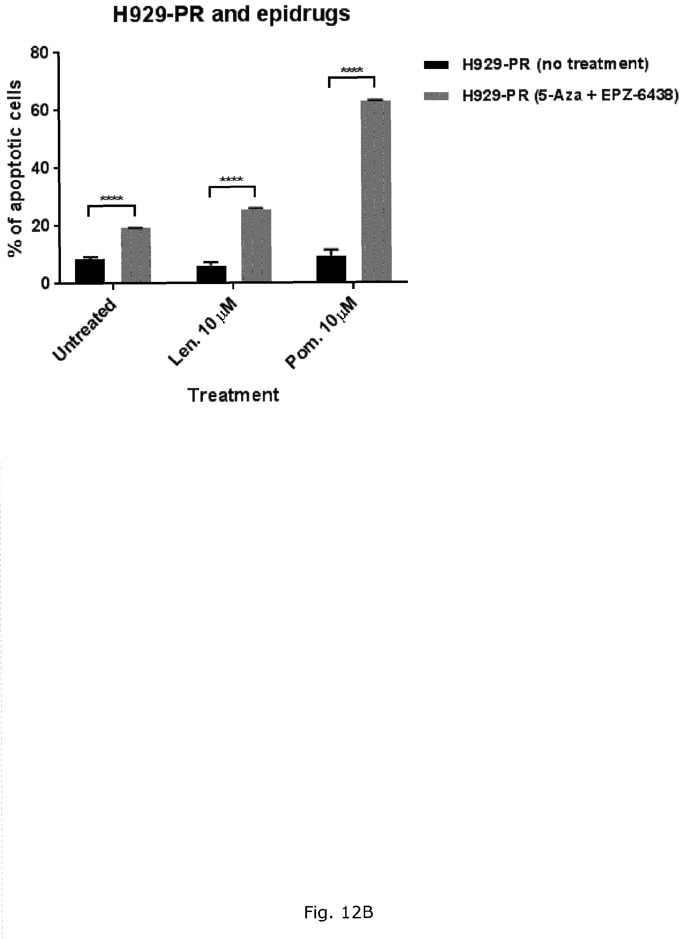

In order to understand whether the epigenetic IMiD-resensitization of cancer cells involved restoration of the epigenetic changes that happened during the developmend of resistance, the present inventors examined the global DNA methylation and chromatin accessibility of the cells after treatment with 5-Azacytidine and EPZ-6438. Even though DNA methylation remained unchanged after the combination therapy, chromatin accessibility was almost entirely restored to the initial state, as shown in figure 11C.

In one embodiment, the epigenetic change that resensitizes the cancer cells to IMiDs is chromatin accessibility.

Finally, the investigators examined whether the combination therapy could overcome intrinsic resistance to IMiDs. By testing two primarily IMiD-resistant cell lines, JJN3 and KMS12-BM, the investigators showed that EPZ-6438 both alone and in combination with 5-Azacytidine were able to render the cells sensitive to both lenalidomide and pomalidomide.

In one embodiment, EPZ-6438 alone can sensitize myeloma cells with intrinsic resistance to IMiDs.

### Combination with Immunomodulatory drug (IMiD)

The present inventors have identified the mechanisms that are responsible for downregulation of CRBN and resistance to IMiDs in cancers such as MM. By reverting this resistance to IMiDs, the present inventors can develop drugs that can restore the sensitivity of the malignant cancer cells to IMiDs.

Thus, in one embodiment the present invention relates to a combination an EZH2 inhibitor and a cytidine analogue further comprising an Immunomodulatory drug (IMiD), preferably Lenalidomide and/or Pomalidomide.

An object of presently preferred embodiments of the present invention relates to the combination of EPZ-6438 and 5-azacytidine, further comprising an Immunomodulatory drug (IMiD), such as but not limited to Lenalidomide and/or Pomalidomide.

Even more interestingly, MDS patients with monosomy 7 (EZH2 is located on chromosome 7) or inactivating mutations of EZH2, have been shown to respond better to treatment with 5-Azacytidine, thus suggesting that dual inhibition of both EZH2 and DNA methyltransferases might be more effective than the latter alone.

### Compositions of the invention

In one embodiment, the present invention relates to a composition for use in treating a cancer, wherein the composition comprises a first component consisting of an effective amount of an EZH2 inhibitor and second component comprising an effective amount of a cytidine analogue.

In some embodiments, the EZH2 inhibitor or first component in the composition of the present invention may be selected from the group consisting of 3-deazaneplanocin A (DZNep), EPZ005687, tazemetostat (EPZ-6438), Sinefungin, EI1, GSK126, and UNC1999.

In some embodiments, the cytidine analogue or second component in the compositions of the present invention may be selected from the group consisting of KP-1461, zebularine, azacitidine and its analog decitabine.

In another embodiment, the present invention relates to a composition for use in treating a cancer, wherein the composition comprises a first component consisting of an effective amount of an EPZ-6438 and second component comprising an effective amount of 5-Azacitidine.

The compositions according to the present invention are effective in the treatment of cancers, wherein the cancer is resistant to Immunomodulatory drugs (IMiDs) chemotherapy.

The compositions according to the present invention are particular useful in the treatment of multiple myeloma.

In one embodiment the compositions are formulated into a pharmaceutical composition comprising an EZH2 inhibitor and a cytidine analogue of the invention, wherein preferably the EZH2 inhibitor is EPZ-6438 and/or the cytidine analogue is 5-Azacytidine.

It should be understood that any combination of the EZH2 inhibitors and the cytidine analogues of the present invention feature as discussed above in the section "Specific combination" apply by analogy to the compositions described herein.

In one embodiment the compositions of the present invention, also comprise a third component such as but not limited an Immunomodulatory drug (IMiD), preferably Lenalidomide and/or Pomalidomide.

### Pharmaceutical compositions

In some embodiments, the compositions are formulated into a pharmaceutical composition. A pharmaceutical composition is a formulation containing a compound in a form suitable for administration to a subject.

A compound disclosed herein and one or more other therapeutic agents described herein each can be formulated individually or in multiple pharmaceutical compositions in any combinations of the active ingredients. Accordingly, one or more administration routes can be properly elected based on the dosage form of each pharmaceutical composition. Alternatively, a compound disclosed herein and one or more other therapeutic agents described herein can be formulated as one pharmaceutical composition.

In another aspect, a composition disclosed herein, or a pharmaceutically acceptable salt, solvate, analogue or derivative thereof, may be administered in combination with radiation therapy.

Radiation therapy can also be administered in combination with a composition disclosed herein and another chemotherapeutic agent described herein as part of a multiple agent therapy.

In one embodiment, the pharmaceutical compositions of the present invention are in bulk or in unit dosage form. The unit dosage form is any of a variety of forms, including, for example, a capsule, an IV bag, a tablet, a single pump on an aerosol inhaler or a vial.

The quantity of active ingredient (e.g., a formulation of the disclosed compound or salt, hydrate, solvate or isomer thereof) in a unit dose of composition is an effective amount and is varied according to the particular treatment involved.

One skilled in the art will appreciate that it is sometimes necessary to make routine variations to the dosage depending on the age and condition of the patient.

As used herein, the phrase "pharmaceutically acceptable" refers to those compounds, anions, cations, materials, compositions, carriers, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

"Pharmaceutically acceptable excipient" means an excipient that is useful in preparing a pharmaceutical composition that is generally safe, non-toxic and neither biologically nor otherwise undesirable, and includes excipient that is acceptable for veterinary use as well as human pharmaceutical use. A "pharmaceutically acceptable excipient" as used herein includes both one and more than one such excipient.

A pharmaceutical composition disclosed herein is formulated to be compatible with its intended route of administration. Examples of routes of administration include parenteral, e.g., intravenous, intradermal, subcutaneous, oral (e.g., inhalation), transdermal (topical), and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

### Medicaments of the invention

The present invention also relates to an effective amount of an EZH2 inhibitor and a cytidine analogue for use in the preparation of a medicament for treating cancer.

In one embodiment the medicament may be used in the treatment of cancer, wherein the cancer is resistant to Immunomodulatory drugs (IMiDs) chemotherapy.

In another embodiment, the medicament functions by an epigenetic mechanism, wherein epigenetic changes in the cancer cells are restored.

In one embodiment, the epigenetic change is chromatin accessibility.

The medicament of the present invention comprises more than one active component. The active component can for example be a chemotherapeutic drug and/or a drug that reverse resistance to the chemotherapeutic drug.

In a preferred embodiment, then one component of the medicaments of the present invention is an EZH2 inhibitor, preferably EPZ-6438.

In another preferred embodiment, then one component of the medicaments of the present invention is a cytidine analogue, preferably 5-Azacytidine.

In one embodiment, then the chemotherapeutic drug of the medicament is an Immunomodulatory drug (IMiD), preferably Lenalidomide and/or Pomalidomide.

### Administration route

The compositions, components or ingredients of the present invention may be jointly or administered separately.

In one embodiment, the compositions or medicaments of the present invention are administered orally (IMiDs, EPZ-6438) and subcutaneously (5-Azacytidine).

The dosage will also depend on the route of administration. A variety of routes are contemplated, including oral, pulmonary, rectal, parenteral, transdermal, subcutaneous, intravenous, intramuscular, intraperitoneal, inhalational, buccal, sublingual, intrapleural, intrathecal, intranasal, and the like. Dosage forms for the topical or transdermal administration of a compound of this invention include powders, sprays, ointments, pastes, creams, lotions, gels, solutions, patches and inhalants. In one embodiment, the active compound is mixed under sterile conditions with a pharmaceutically acceptable carrier, and with any preservatives, buffers, or propellants that are required.

### Cancer

Cancer is characterized primarily by an increase in the number of abnormal cells derived from a given normal tissue, invasion of adjacent tissues by these abnormal cells, and lymphatic or blood-borne spread of malignant cells to regional lymph nodes and to distant sites (metastasis).

The present invention pertains to cancers such as lung cancer, multiple myeloma, lymphoma and epithelial ovarian cancer.

In one embodiment, the cancer is multiple myeloma.

In the present context, Multiple myeloma, also known as plasma cell myeloma, is a cancer of plasma cells, a type of white blood cell, which represents the final stage of differentiated B cells and is able to produce antibodies.
Multiple myeloma (MM) is thus a malignant disease characterized by proliferation of plasma cells in the bone marrow. It is thought to arise by clonal expansion of plasma cells, initially leading to a condition known as MGUS, that can later evolve to the so-called "smouldering" myeloma, and finally to symptomatic MM. Symptomatic MM consists of, apart from the presence of a clonal immunoglobulin in the serum and/or urine, end-organ damage, presenting as anemia, hypercalcemia, diminished renal function, and bone disease.

Although morphologically similar, MM is clinically heterogeneous due to a complex pathobiology. The use of FISH (fluorescence in-situ hybridization) has revealed specific cytogenetical abnormalities, such as t(4;14) or del(17p), which are related to a worse prognosis, and FISH is now used as a standard tool for biological and prognostic classification of MM. Apart from the genetic abnormalities that characterize MM, epigenetic aberrations, such as abnormal DNA methylation and histone modifications, or abnormal miRNA expression, have also been shown to be a part of the disease's biology. Some of the genome-wide mutations detected in MM affect enzymes that regulate the epigenome and status of chromatin. The introduction of high-dose chemotherapy with autologous stem-cell support, as well as novel, drugs, such as bortezomib or "IMiDs" (thalidomide, lenalidomide, pomalidomide), has improved the survival of patients with MM, but the disease is still considered incurable with the current treatment modalities.

A subject in need thereof may have refractory or resistant cancer. "Refractory or resistant cancer" means cancer that does not respond to treatment. The cancer may be resistant at the beginning of treatment or it may become resistant during treatment.

In some embodiments, the subject in need thereof has cancer recurrence following remission on most recent therapy.

In some embodiments, the subject in need thereof received and failed all known effective therapies for cancer treatment.

In some embodiments, the subject in need thereof has received at least one prior therapy.

In certain embodiments, the prior therapy is monotherapy.

In certain embodiments, the prior therapy is combination therapy.

### Methods of the invention

The present invention also relates to any methods of treatment derived from the scientific contribution to the art provided by the present disclosure, such as but not limited to a method for treating cancer comprising administering a combination of an EZH2 inhibitor and a cytidine analogue to a patient in need thereof.

Such treatment regimens are in particular addressing the resensitization of IMiDs, and thus in one embodiment, the present invention relates to a method for reversing drug resistance in a patient having an Immunomodulatory drug (IMiD) resistant cancer comprising administering to said patient a combination of an EZH2 inhibitor and a cytidine analogue.

In another embodiment, the invention relates to a method for treating cancer comprising administering to a subject in need thereof
(a) an effective amount of an EZH2 inhibitor, and
(b) an effective amount of a cytidine analogue
to provide a combination therapy having an enhanced therapeutic effect compared to the effect of the EZH2 inhibitor and the cytidine analogue administered alone.

In a preferred embodiment, the combination therapy has a synergistic therapeutic effect.

As shown in the EXAMPLES, then the method is particularly useful when the method further comprises administering at least one additional anti-cancer agent.

In a presently preferred embodiment, said additional anti-cancer agent is an immunomodulatory drug (IMiD), preferably Lenalidomide or Pomalidomide or a combination thereof.

In another embodiment, the methods of the present invention is particularly useful when the cancer is resistant to Immunomodulatory drugs (IMiDs) chemotherapy.

The mechanism through which the methods of the present invention function, is when the EZH2 inhibitor and a cytidine analogue restore the epigenetic changes in the cancer cells originally contribution to the IMiD resistance.

In one embodiment, said epigenetic change is chromatin accessibility.

In a preferred embodiment, the method of treatments of the present invention provides means for treating cancer, especially wherein the cancer is multiple myeloma.

In a preferred embodiment the EZH2 inhibitor used in the methods according to the present invention is EPZ-6438.

In a preferred embodiment the cytidine analogue used in the methods according to the present invention is 5-Azacytidine.

### Kit of parts

In combination therapy, the therapeutic benefit relates to taking two or more different pharmaceutical actives together.

The therapeutic benefit can be a synergistic or inter-working relationship between the actives in the patient. Thus, the present invention also relates to a preparation in the form of a "kit-of-parts" in which the individual active compounds, are physically separated, and includes instructions to the use of those compounds, either simultaneously, separately or sequentially, because as disclosed herein they produce a new and unexpected joint therapeutic effect, which cannot be attained by the compounds independently of each other.

Thus, in one embodiment the present invention relates to a kit for treating cancer comprising
(a) an effective amount of an EZH2 inhibitor and
(b) an effective amount of a cytidine analogue, and
(c) optionally instruction for use thereof.

In a further embodiment, said kit also comprise an effective amount of an IMiD.

The instructions provide instruction for simultaneous, concurrent, separate or sequential use of the effective amounts of the EZH2 inhibitor, the cytidine analogue and/or the IMiD.

### Combination with further chemotherapeutics

Combination therapies are being studied as possible treatments when primary treatments begin to fail. Etoposide, a topoisomerase inhibitor, when combined with an EZH2 inhibitor, becomes more effective for non-small cell lung cancers with BRG1 and EGFR mutations.

Even more interestingly, EZH2 inhibition using EPZ-6438 can potentially also resensitize myeloma cells to the proteasome inhibitor bortezomib, which is another essential drug for the treatment for MM

### Dose

In one embodiment, EPZ-6438 or a pharmaceutically acceptable salt thereof is administered to the subject at a dose of approximately 100 mg to approximately 3200 mg daily, such as 100 mg BID to 1600mg BID (e.g., 100 mg BID, 200 mg BID, 400 mg BID, 800 mg BID, or 1600 mg BID), for treating a MM.

On one embodiment the dose is 400 to 1200 mg BID, preferably 800 mg BID.

The standard dose of Lenalidomide, as used in the clinic, is 25 mg once per day, on days 1-21 on a 28-day cycle (combined with Dexamethasone).

Thus, in one embodiment, Lenalidomide or a pharmaceutically acceptable salt thereof is administered to the subject at a dose of approximately 1 mg to approximately 100 mg daily, such as 10 mg BID to 50mg BID (e.g., 10 mg BID, 20 mg BID, 40 mg BID, 80 mg BID, or 90 mg BID), for treating a MM.

The standard dose of Pomalidomide, as used in the clinic, is 4 mg once per day, on days 1-21 on a 28-day cycle (combined with Dexamethasone)

### Dose

### Dose of EPZ-6438

In one embodiment, EPZ-6438 or a pharmaceutically acceptable salt thereof is administered to the subject at a dose of 100 mg to 3200 mg daily, such as 100 mg BID to 1600mg BID e.g., 100 mg BID, 200 mg BID, 400 mg BID, 800 mg BID, or 1600 mg BID, for treating MM.

In one embodiment, EPZ-6438 or a pharmaceutically acceptable salt thereof is administered to the subject at a dose of 400 mg to 1200 mg daily

In one embodiment, EPZ-6438 or a pharmaceutically acceptable salt thereof is administered to the subject at a dose of 600 mg to 1000 mg daily

In one embodiment, EPZ-6438 or a pharmaceutically acceptable salt thereof is administered to the subject at a dose of 700 mg to 900 mg daily

In one embodiment, the dose is 800 mg BID.

### Dose of Lenalidomide

The standard dose of Lenalidomide, as used in the clinic, is 25 mg once per day, on days 1-21 on a 28-day cycle - typically combined with Dexamethasone.

Thus, one embodiment of the present invention relates to a dose of Lenalidomide of 1-50 mg per day.

Thus, one embodiment of the present invention relates to a dose of Lenalidomide of 10-40 mg per day.

Thus, one embodiment of the present invention relates to a dose of Lenalidomide of 20-30 mg per day.

In one embodiment, the dose is 25 mg per day.

### Dose of Pomalidomide

The standard dose of Pomalidomide, as used in the clinic, is 4 mg once per day, on days 1-21 on a 28-day cycle - typically combined with Dexamethasone.

Thus, one embodiment of the present invention relates to a dose of Pomalidomide of 0,5-10 mg per day.

Thus, one embodiment of the present invention relates to a dose of Pomalidomide of 1-8 mg per day.

Thus, one embodiment of the present invention relates to a dose of Pomalidomide of 2-6 mg per day.

In one embodiment, the dose of Pomalidomide is 4 mg per day.

### Dose of 5-Azacitidine (Vidaza)

The standard dose of 5-Azacitidine (Vidaza), as used in the clinic, is 75 mg/m² for 5 days, in a 28-day cycle.

Thus, one embodiment of the present invention relates to a dose of 5-Azacitidine (Vidaza)of 25-150 mg/m² for 5 days, in a 28-day cycle.

Thus, one embodiment of the present invention relates to a dose of 5-Azacitidine (Vidaza)of 50-100 mg/m² for 5 days, in a 28-day cycle.

Thus, one embodiment of the present invention relates to a dose of 5-Azacitidine (Vidaza)of 60-80 mg/m² for 5 days, in a 28-day cycle.

In one embodiment, the dose of 5-Azacitidine (Vidaza)is 75 mg/m² for 5 days, in a 28-day cycle.

### General

It should further be understood that the doses discussed above for the exemplified species according to the invention applies by analogy to the genus's for the compositions, medicaments, and components described herein.

It should be understood that any feature and/or aspect discussed above in connections with the compounds according to the invention apply by analogy to the methods described herein.

It should further be understood that any feature and/or aspect discussed above in connection with the combination of the invention according to the invention applies by analogy to the compositions, medicaments and uses described herein.

The terms *cytidine analogue* and *DNA methyltransferase inhibitors,* are used interchangeably.

The following figures and examples are provided below to illustrate the present invention. They are intended to be illustrative and are not to be construed as limiting in any way.

### BRIEF DESCRIPTION OF THE FIGURES

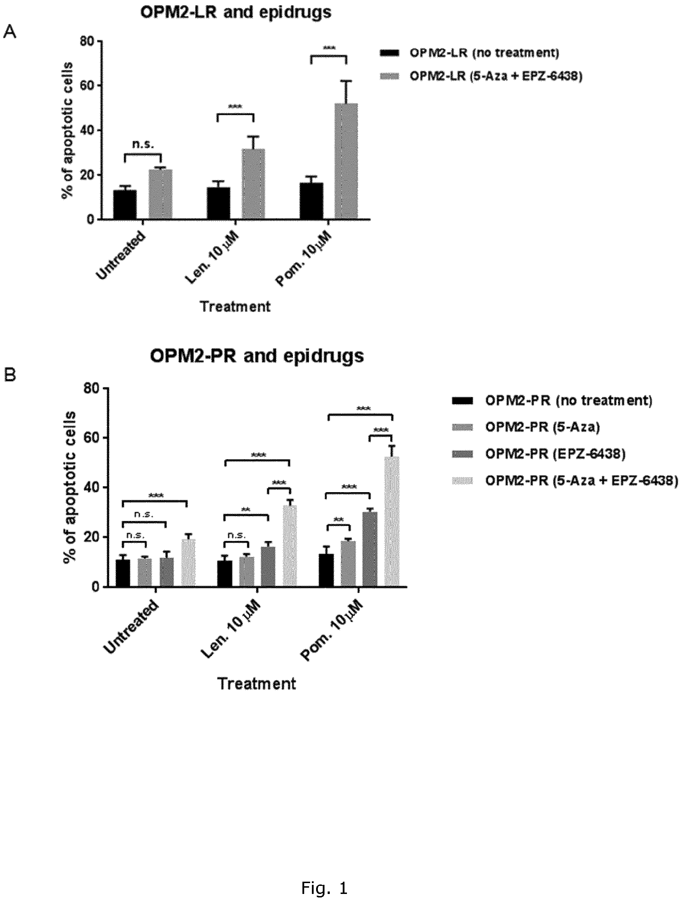

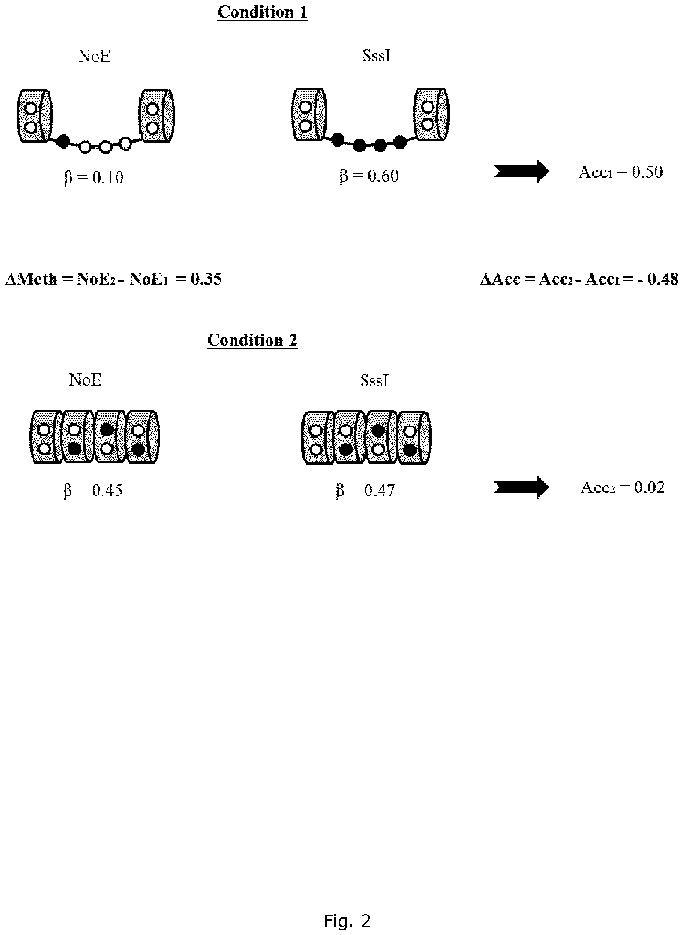

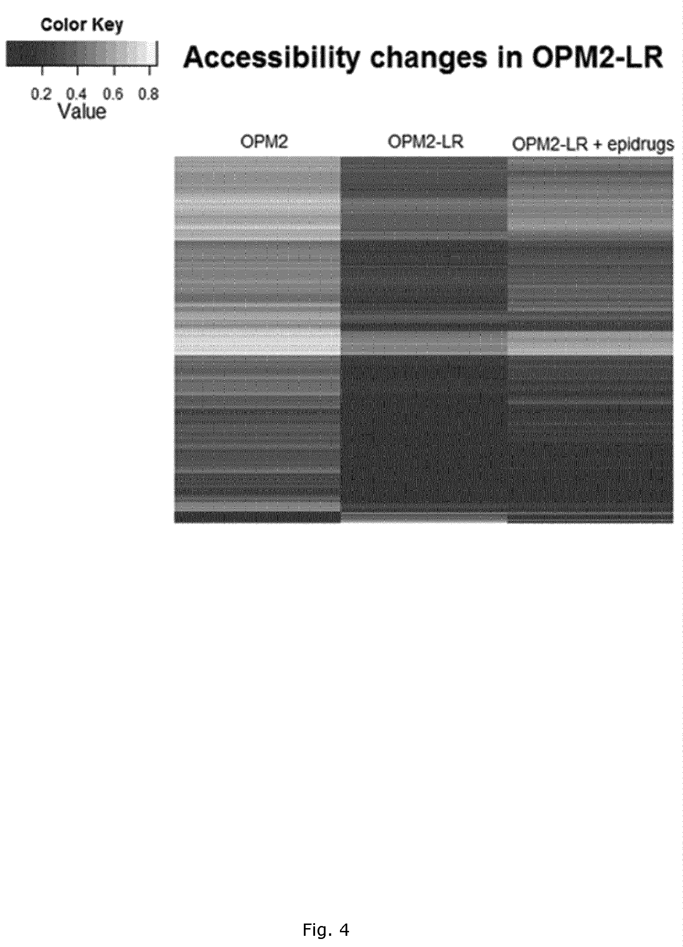

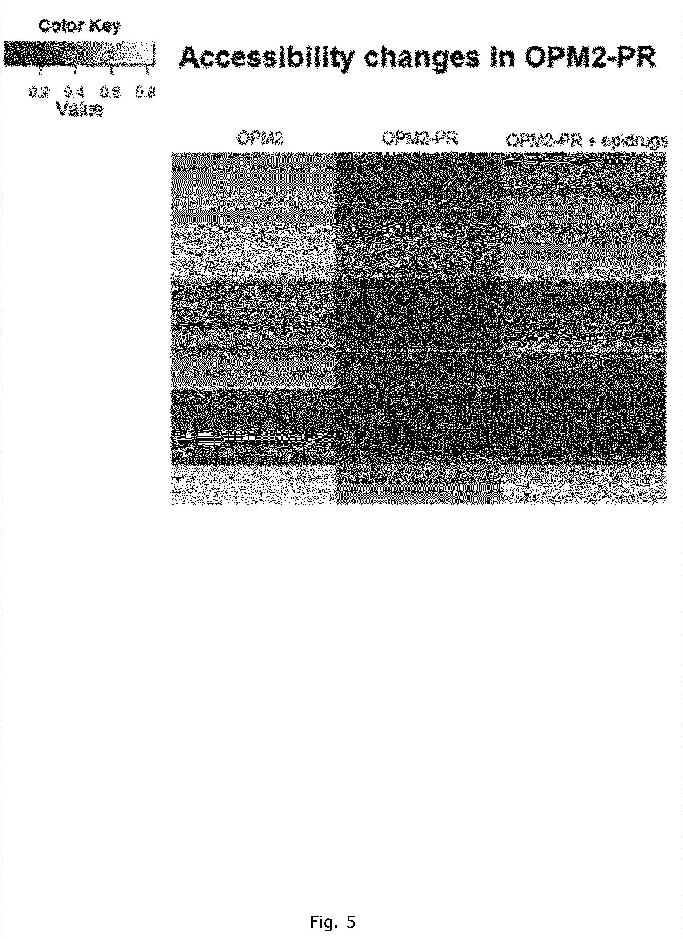

***Figure 1***
   Apoptosis assay for (A) OPM2-LR and (B) comparing no treatment (black bars) vs pretreatment with the combination of 5-azacytidine (0,1 µM) and EPZ-6438 (10 µM) for 48 hours. The cells were treated with nothing (control), 10 µM Lenalidomide or 10 µM Pomalidomide and the apoptotic cells were measured after 3 days of treatment. For OPM2-PR, *the present inventors* also compared the effects of monotherapy with 5-Azacytidine and EPZ-6438 in resensitization to IMiDs. The figures show a significant increase of apoptotic cells after treatment with the epidrugs combination prior to treatment with either lenalidomide or pomalidomide.
***Figure 2***
   Principle of the Acce*SssI*ble assay. For each cell line, there are two different conditions: a no-enzyme treatment (NoE) and a M.SssI-treated one. The enzyme will methylate all the CpG sites genome-wide, as long as they are not covered by a nucleosome. Thus, the substraction of NoE from SSSI will give the accessibility values for each cell line. The difference of accessibility between two different cell lines (ΔAccess) can then be calculated by subsequent substraction of their accessibility values for each probe, while the difference of methylation (ΔMeth) can be calculated by the substraction of NoE values for each cell line.
***Figure 3***
   Density scatterplot comparing the global methylation changes (y axis) and global chromatin accessibility changes (x axis) in OPM2-PR compared to OPM2, as measured with Acce*SssI*ble. Similar to figure 2, the acquirement of pomalidomide resistance in OPM2 is associated with a shift towards lower chromatin accessibility
***Figure 4***
   Heatmap with all the probes showing changes in chromatin accessibility when comparing OPM2-LR to OPM2. The first two columns show that the majority of probes change to a lower chromatin accessibility upon acquired lenalidomide-resistance in OPM2, but treatment with 5-Azacytidine and EPZ-6438 (epidrugs) can almost entirely restore the chromatin accessibility to the initial state.
***Figure 5***
   Heatmap similar to figure 4, but for OPM2-PR. Again, it is clear that treatment with epidrugs can reverse the chromatin accessibility to the initial levels, when the cells were IMiD-sensitive.
***Figure 6***
   Density scatterplot comparing the global methylation changes (y axis) and global chromatin accessibility changes (x axis) in OPM2-LR compared to OPM2, as measured with Acce*SssI*ble. The cutoff for every significant change was set to ± 0.2 (continuous white lines). There is a thus clear tendency towards lower chromatin accessibility upon acquirement of lenalidomide resistance in OPM2 cells.
***Figure 7***
   Dose response curves for (A) OPM2, OPM2-LR (Lenalidomide-resistant) and OPM2-PR (Pomalidomide-resistant) and (B) NCI-H929, NCI-H929-LR (Lenalidomide-resistant) and NCI-H929-PR (Pomalidomide-resistant)
***Figure 8***
   (A) CRBN expression at mRNA level assessed by qPCR. CRBN is significantly downregulated upon acquired resistance to both Lenalidomide and Pomalidomide in both cell lines (OPM2, NCI-H929). (B) Cytospin and immunohistochemistry for CRBN for NCI-H929 and its lenalidomide-resistant counterpart (NCI-H929-LR), showing a reduction of CRBN expression at protein level at NCI-H929-LR
***Figure 9***
   (A): Density scatterplot with the methylation changes (y axis) versus chromatin accessibility changes (x axis) between OPM2 and OPM2-PR. Acquired resistance to pomalidomide is associated with a shift towards decreased accessibility.
   (B): Heatmaps with all the probes exhibiting methylation (green/red) and accessibility changes (yellow/blue) in OPM2-PR.
   (C) Genomic location of the probes with significant changes, based on annotation by Illumina. The majority of changes in promoters and enhancers involve chromatin accessibility, while the methylation changes occur mainly in gene bodies and intergenic regions
***Figure 10***
   Methylation curve analysis for the promoter of CRBN in (A) OPM2, NCI-H929 and their IMiD-resistant counterparts, (B) CD138+ cells from a cohort of 41 newly diagnosed MM patients and (C) CD138+ cells from a cohort of 48 relapsed myeloma patients. The blue lines represent the methylation controls (right: unmethylated control, left: methylated control), while the green lines represent the samples analyzed. None of the cell lines or patient samples showed signs of DNA methylation in the promoter area of CRBN.
***Figure 11***
   (A) Density scatterplot with methylation and accessibility changes in OPM2-PR treated with 0,1 µM 5-Aza and 10 µM EPZ-6843 for 48 hours. There are markedly less probes exhibiting low accessibility (below -0,2) compared to OPM2-PR (figure 5A).
   (B) Apoptosis results for OPM2-PR comparing no treatment and treatment with 5-Aza, EPZ-6438 or combination treatment for 48 hours, followed by treatment with nothing, 10 µM lenalidomide or 10 µM Pomalidomide for 3 days. The error bars represent SD. Each experiment was performed as a duplicate and at least two times independently. Even though monotherapy with EPZ-6438 has a minor resensitization effect, the combination treatment resensitizes the cells much more significantly to both lenalidomide and pomalidomide.
   (C) Heatmaps with all the probes showing significant accessibility (blue/green) and methylation (red/green) in OPM2-PR. The treatment with 5-Aza and EPZ-6438 restores accessibility in the majority of probes, but fails to restore normal methylation levels.
***Figure 12***
   Epigenetic resensitization of (A) H929-LR and (B) H929-PR after treatment with 0,5 µM 5-Aza combined with 10 µM EPZ-6438 for 48 hours.
***Figure 13***
   A) CRBN expression assessed by qPCR in IMiD-resistant OPM2 and treatment with epidrugs. The expression of CRBN remained unchanged despite resensitization of cells to IMiDs.
   (B) ChIP data for the area around the transcription site of CRBN, showing no presence of H3K27me3.
   (C) Apoptosis data showing epigenetic sensitization of JJN3 and KMS12-BM, both intrinsically resistant to IMID.

### EXAMPLES

### Example 1 - Cell culture and treatment

A total of seven commercially available human myeloma cell lines (JJN3, OPM2, RPMI-8226, U266, NCI-H929, LP-1, KMS12-BM) were purchased from DSMZ (Leibniz Institute DSMZ-German Collection of Microorganisms and Cell Cultures, Braunschweig, Germany).

The two most IMiD-sensitive cell lines (NCI-H929 and OPM2) were continuously cultured with escalating doses of Lenalidomide and Pomalidomide (Selleck Chemicals) for 4-6 months, until achievement of stable resistance.

Cell proliferation (and IC50 for each cell line) was assessed by measuring 2,3-Bis-(2-Methoxy-4-Nitro-5-Sulfophenyl)-2H-Tetrazolium-5-Carboxanilide (XTT) dye absorbance 3 days after treatment with IMiDs. Cell apoptosis was evaluated using Annexin V and PI, with the analysis performed on a FACS-Calibur (BD Bioscience).

### Example 2 - Nucleosome positioning (AcceSssIble assay)

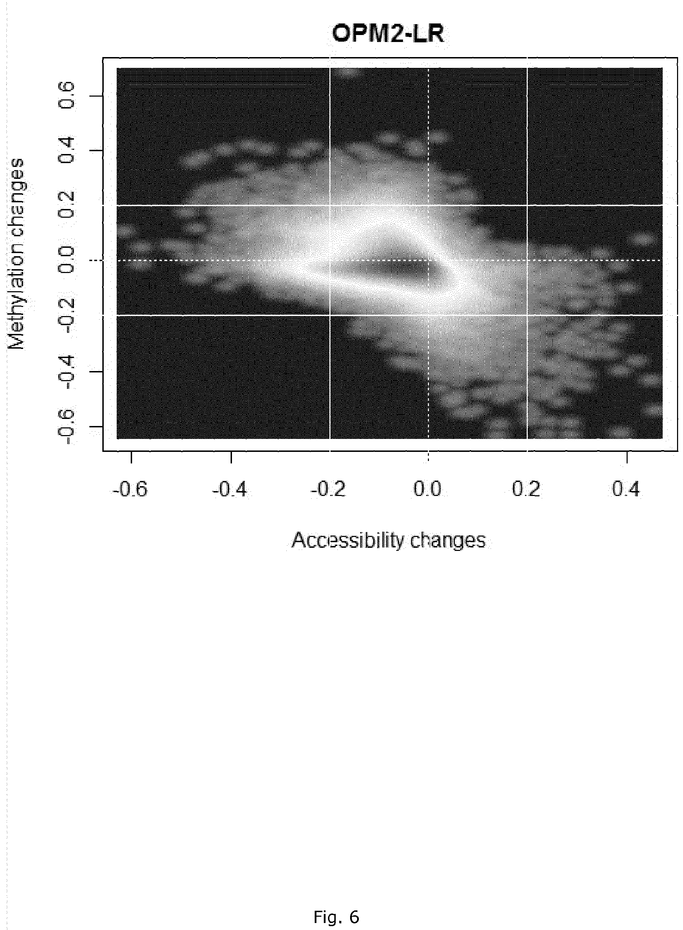

Cell nuclei are isolated and treated with either M.SssI or a no-enzyme treatment control - NoE), DNA is extracted, bisulfite converted and assayed on the Illumina methylation 850K (EPIC) beadchip. Endogenous DNA methylation status is obtained from the no-enzyme control (NoE), and information on chromatin state for unmethylated probes is obtained from the accessibility of the M.SssI methyltransferase to CpG dinucleotides (SSSI) (see figure 6 and figure 2).

### Example 3- CRBN is downregulated in acquired IMiD resistance

First, the present inventors tested the sensitivity of all HMCL to both lenalidomide and pomalidomide by generating dose-response curves and calculating the IC₅₀ of both drugs for each cell line (given in tables below).

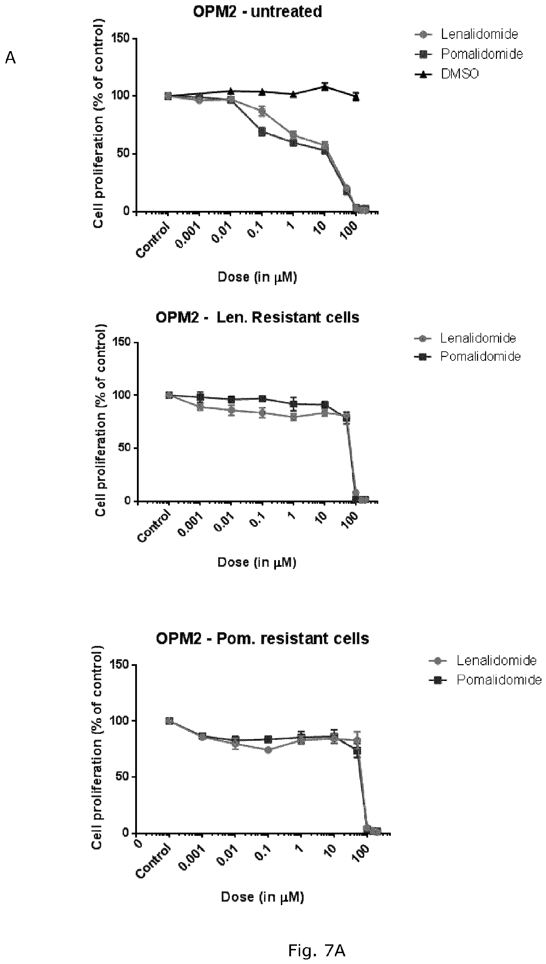

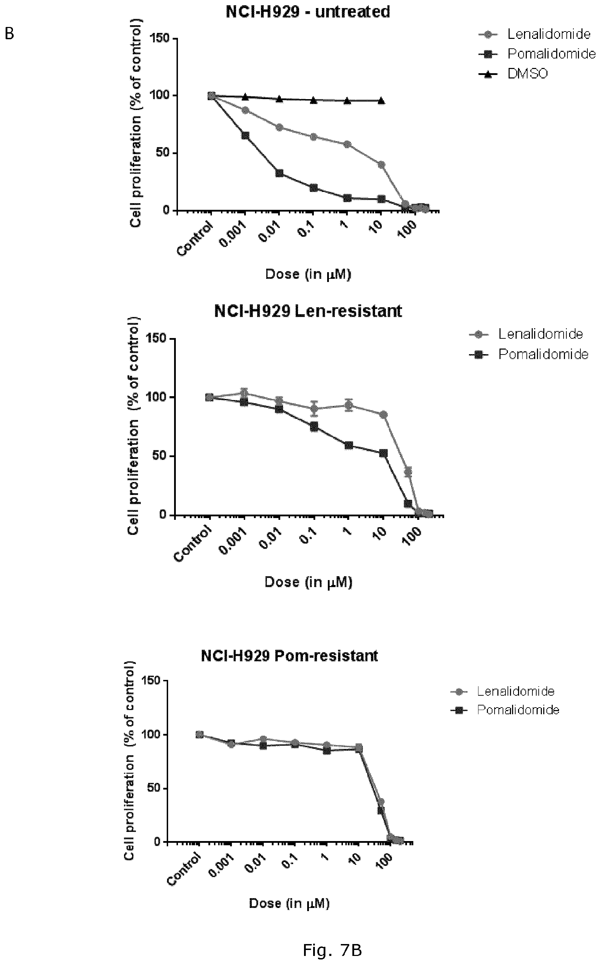

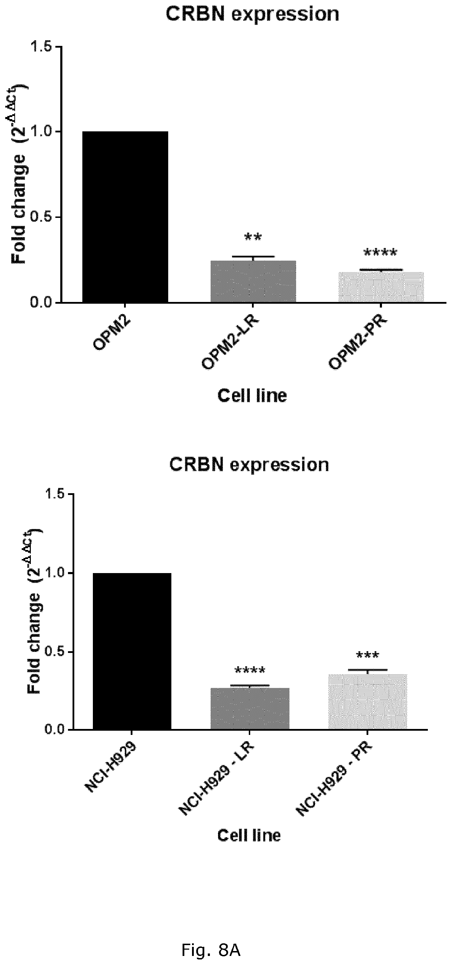

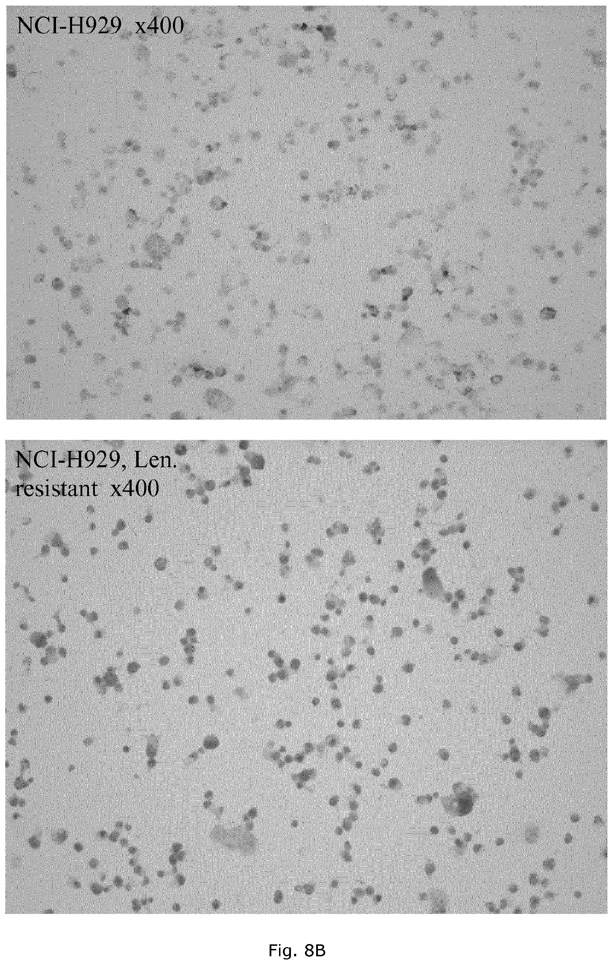

Using the two most IMiD-sensitive cell lines, namely OPM2 and NCI-H929, the present inventors were able to generate lenalidomide- and pomalidomide-resistant cell lines (OPM2-LR, OPM2-PR and NCI-H929-LR, NCI-H929-PR, respectively) by continuously culturing the cells in the presence of IMiDs (figure 7). Consistent with previously published data, the present inventors observed a significant reduction of CRBN expression in all the resistant cell lines when compared to their sensitive counterpart, both at mRNA and at protein level (Figure 8).

### Example 4 - CRBN is not regulated by promoter DNA methylation

Mutations in CRBN are rare therefore the present inventors showed that epigenetic silencing through promoter hypermethylation is a mechanism explaining the low expression of CRBN in the IMiD-resistant cell lines.

Using methylation-specific melting curve analysis, the present inventors showed that all the IMiD-sensitive and resistant cell lines, as well as a total of 48 patients with newly diagnosed MM and 41 patients with relapsed MM.

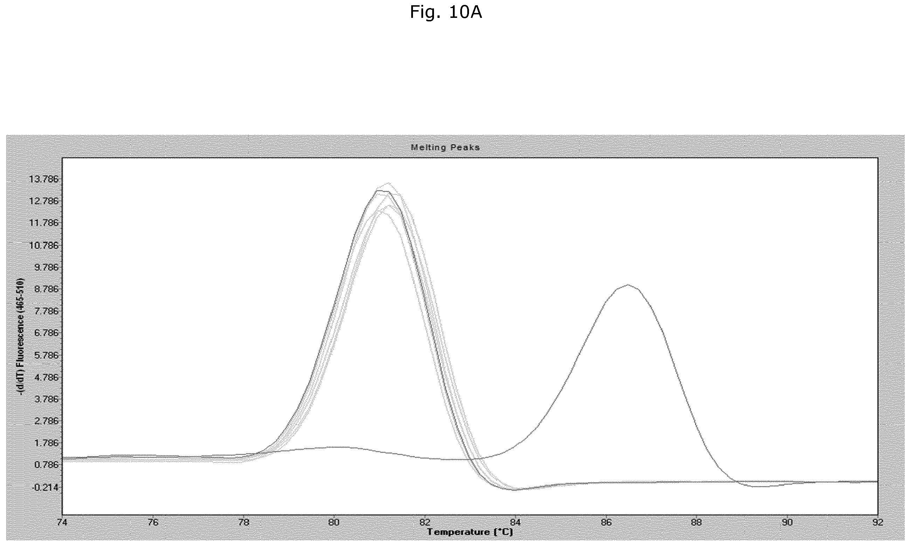

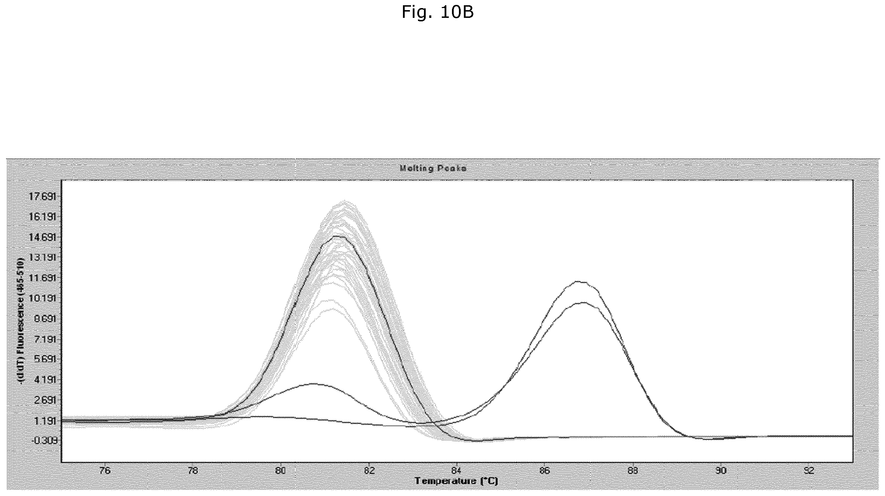

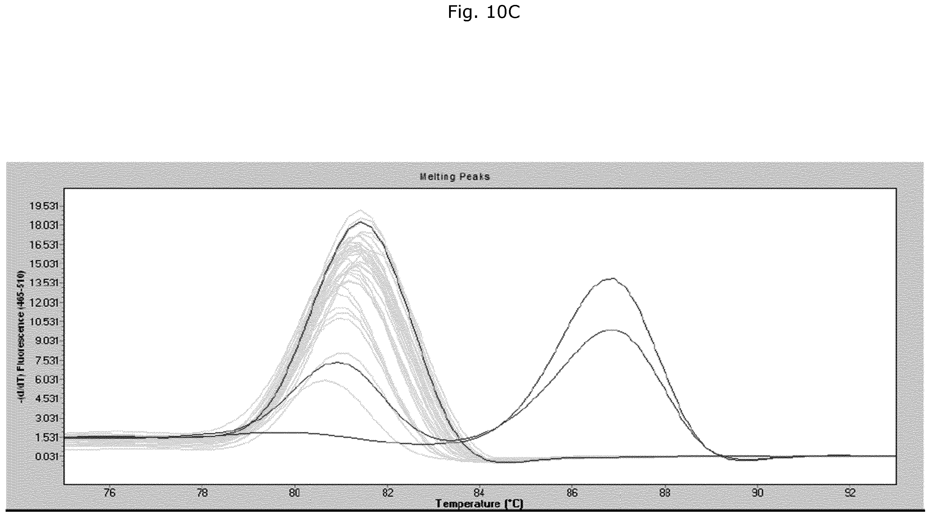

Interestingly, none of the cell lines (figure 10A), and none of the patient samples (figure 10B, 10C) showed hypermethylation of the promoter area of CRBN.

Overall, these data show that the promoter of *CRBN* is completely unmethylated, and variations in its expression are not due to epigenetic regulation through DNA methylation.

### Example 5 - Acquired IMiD resistance is associated with decreased chromatin accessibility (increased nucleosome occupancy)

After having excluded DNA methylation as a potential epigenetic regulatory mechanism of CRBN expression, the present inventors went on to examine whether the promoter of CRBN exhibited altered nucleosome positioning in the IMiD-resistant cell lines.

Using Acce*SssI*ble, the present inventors compared both the global DNA methylation and chromatin accessibility changes in the IMiD-sensitive vs. IMiD-resistant cell lines, and found that acquired resistance to IMiDs is primarily associated with a global decrease in chromatin accessibility rather than global methylation changes. (See Figures 2,3, 9A, 9B).

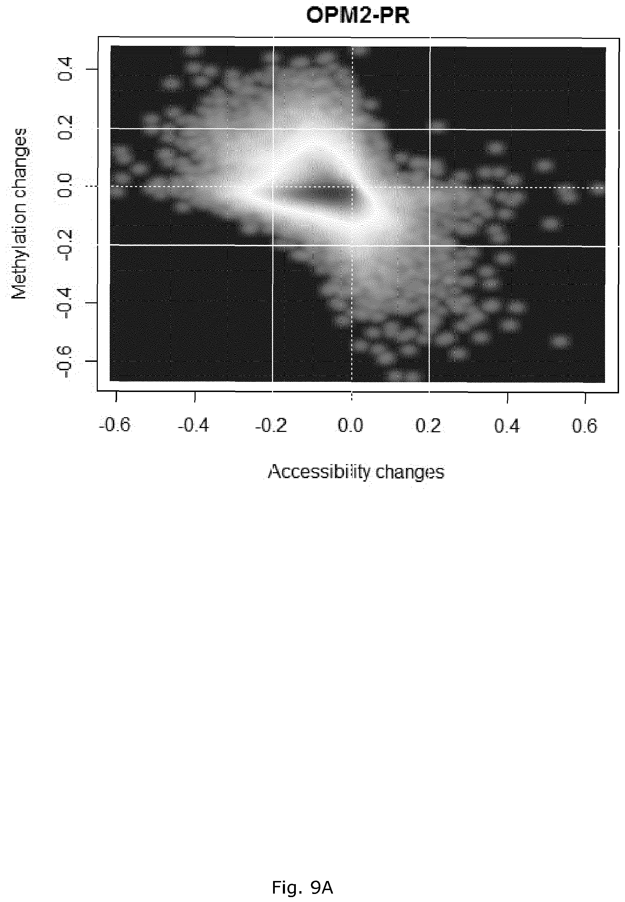

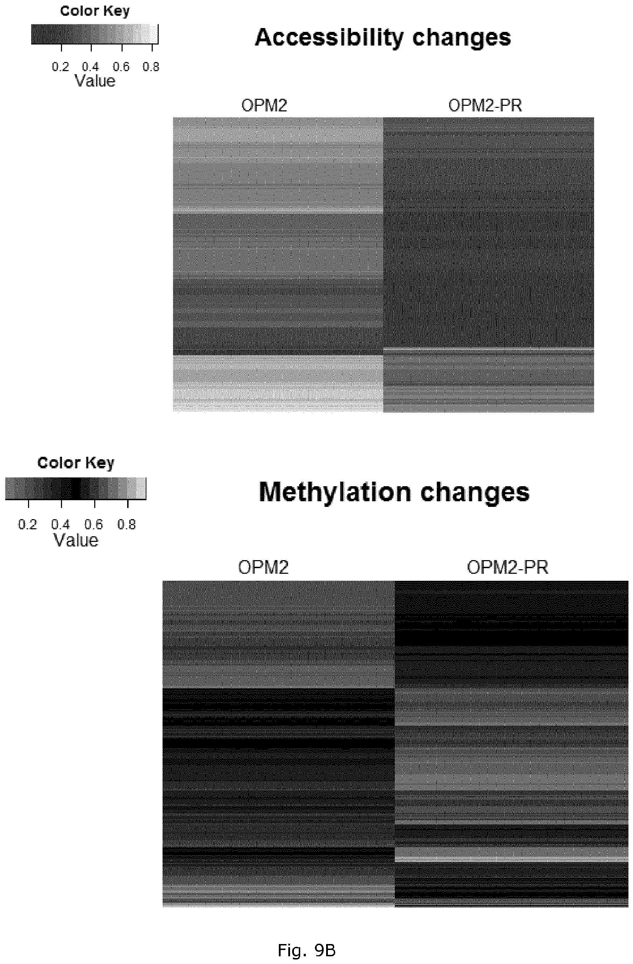

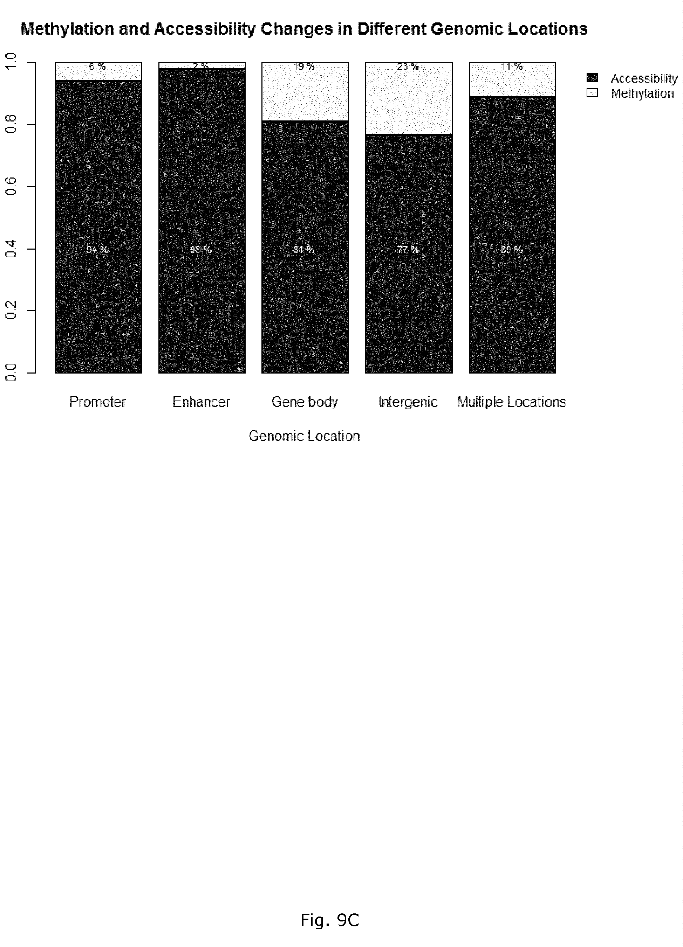

In addition, most of the changes in nucleosome positioning occurred in gene promoters or enhancers, while methylation changes mainly occurred in gene bodies or intergenic regions (figure 9C)

### Example 6 - Acquired IMiD resistance reversible with epitherapy

Since nucleosome positioning (and H3K37me3) has been shown to be a reversible epigenetic mechanism, the present inventors decided to examine whether it was possible to restore accessibility of important regulatory genomic areas by treatment with epigenetic drugs, such as DNA methyltransferase inhibitors (5-Azacytidine), HDAC inhibitors (Panobinostat), and EZH2 inhibitors (EPZ-6438), both as monotherapy and as combination therapy.

The present inventors treated all IMiD-resistant cell lines with different epidrugs for 48 hours and then exposed them to IMiDs (no treatment vs. 10 µM lenalidomide or pomalidomide), measuring their sensitivity by apoptosis and cell cycle assays.

Pretreatment with panobinostat failed to resensitize the cells to IMiDs either as monotherapy or combined with 5-Azacytidine or EPZ-6438 (data not shown).

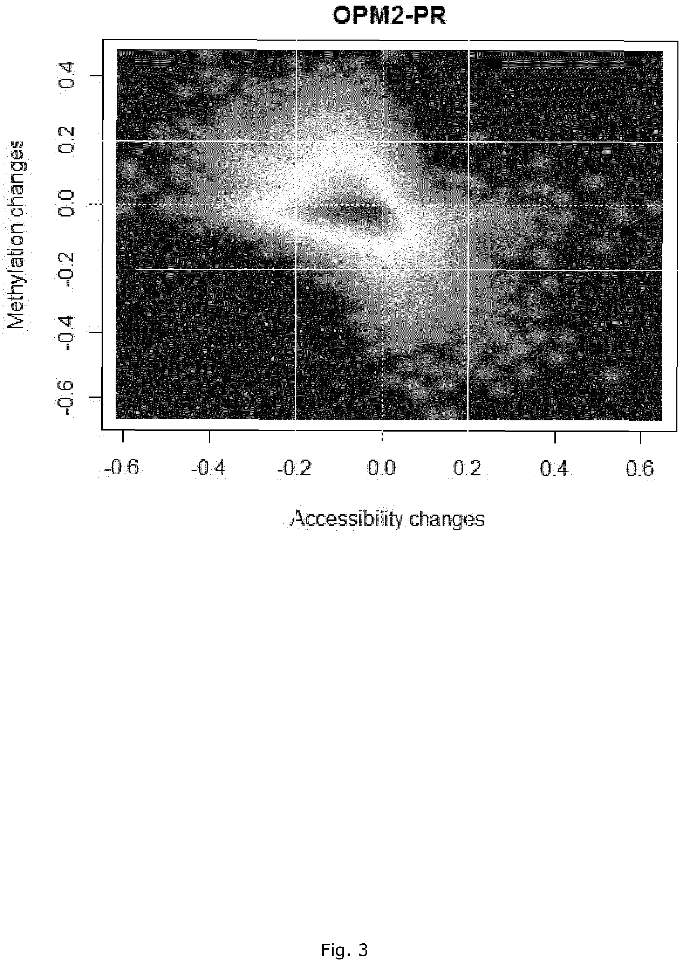

Similarly, monotherapy with either 5-Azacytidine or EPZ-6438 did not restore IMiD sensitivity; however, after exposure to a combination of 5-Azacytidine and EPZ-6438 for 48 hours, resistant cells showed significantly increased sensitivity to both lenalidomide and pomalidomide, through an apoptosis-inducing mechanism (Figure 3C, 3D).

These data show that acquired resistance to IMiDs can potentially be overcome by reprogramming malignant plasma cells with a combination of epigenetic drugs.

### Example 7 - Epigenetic IMiD resensitization is CRBN-independent and potentially effective in intrinsic IMiD resistance

As shown by our previous results, CRBN is not regulated by either promoter DNA methylation or nucleosome occupancy. Since EZH2 inhibition was shown to resensitize the cells to IMiDs, the present inventors went on to look whether this effect was associated with an increase of CRBN expression.

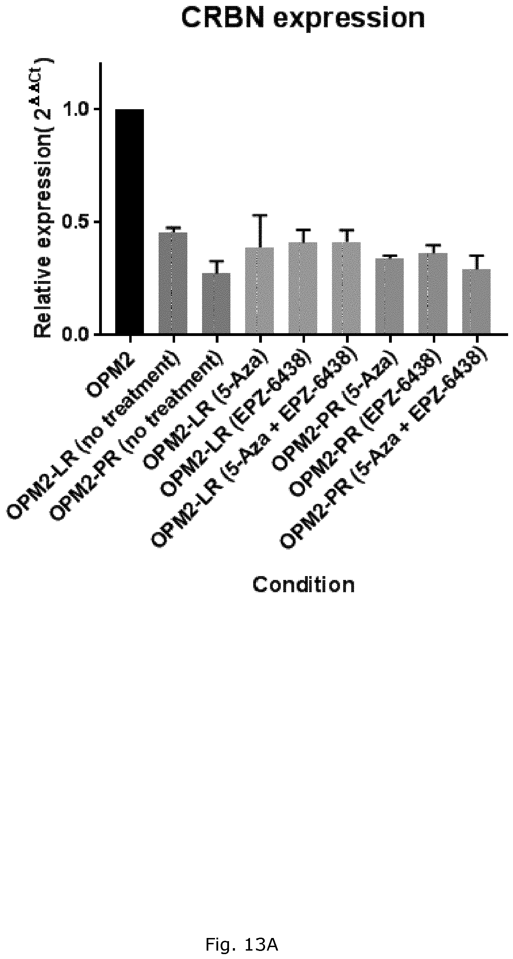

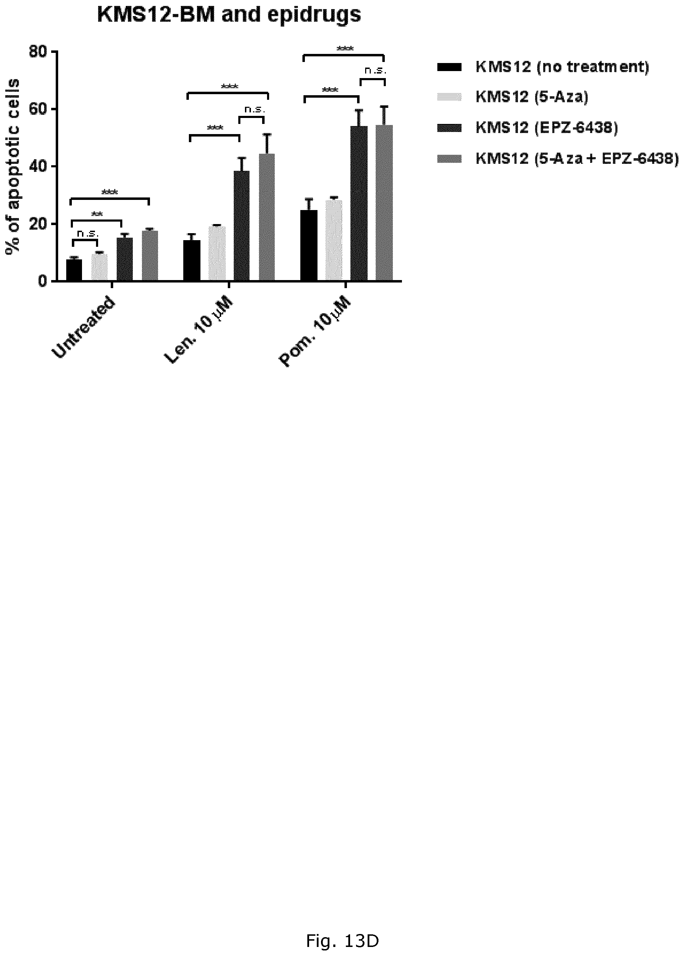

By performing qPCR, the present inventors showed that the expression of CRBN remained low and unchanged in both OPM2-LR and OPM2-PR despite the treatment with 5-Azacytidine and EPZ-6438 (both as monotherapy and in combination), disclosing a CRBN-independent mechanism of resensitization to IMiDs (Figure 13A).

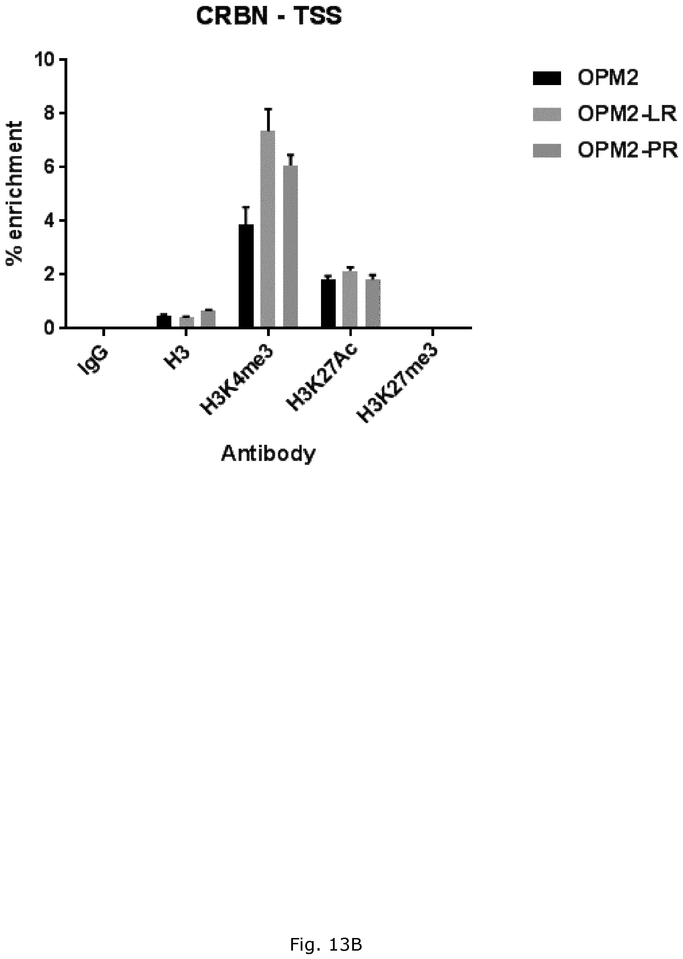

Supporting this finding, the present inventors did not detect any H3K27me3 histone marks on the promoter area of CRBN (Figure 13B).

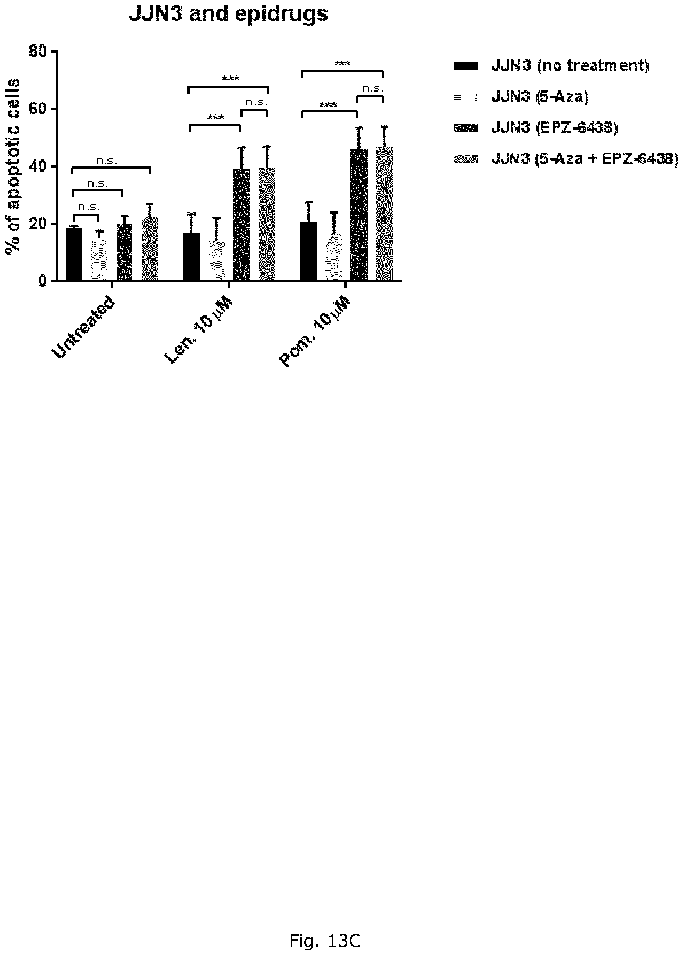

The present inventors then went on to examine whether the combination of epidrugs was effective in intrinsically IMiD-resistant cell lines. Interestingly, the present inventors showed that for two of the cell lines with baseline IMiD resistance (JJN3 and KMS12-BM), both EPZ-6438 as a monotherapy and in combination with 5-Azacytidine were able to render the cells sensitive to both lenalidomide and pomalidomide (Figure 13C), showing that resistance to IMiDs, no matter if it is intrinsic or acquired, is potentially reversible, and CRBN-independent.

**Data table**

| **Cell line** | **IC₅₀ for Lenalidomide** | **IC₅₀ for Pomalidomide** |
|---|---|---|
| *JJN3* | 135,6 µM | 87,34 µM |
| *OPM2* | 5,012 µM | 2,006 µM |
| *RPMI* | 117,5 µM | 98,33 µM |
| *U266* | 68,11 µM | 60,47 µM |
| *KMS12*-*BM* | 20,81 µM | 15,22 µM |
| *NCI-H929* | 0,563 µM | 0,003 µM |

### ITEMS OF THE INVENTION

1) A combination of an EZH2 inhibitor and a cytidine analogue for use in the treatment in cancer.
2) A combination according to item 1, wherein the cancer is resistant to Immunomodulatory drugs (IMiDs) chemotherapy.
3) A combination according to any of items 1-2, wherein epigenetic changes in the cancer cells are restored.
4) A combination according to any of items 1-3, wherein the epigenetic change is chromatin accessibility.
5) A combination according to any of items 1-4, wherein the cancer is multiple myeloma.
6) A combination according to any of items 1-5, wherein the EZH2 inhibitor is EPZ-6438.
7) A combination according to any of items 1-6, wherein in the cytidine analogue is 5-Azacytidine.
8) A combination according to any of items 1-7, further comprising an Immunomodulatory drug (IMiD), preferably Lenalidomide and/or Pomalidomide.
9) A composition for use in treating a cancer, the composition comprising a first component consisting of an effective amount of an EZH2 inhibitor and second component comprising an effective amount of a cytidine analogue.
10) A composition according to item 9, wherein the cancer is resistant to Immunomodulatory drugs (IMiDs) chemotherapy.
11) A composition according to any of items 9-10, wherein epigenetic changes in the cancer cells are restored.
12) A composition according to any of items 9-11, wherein the epigenetic change is chromatin accessibility.
13) A composition according to any of items 9-12, wherein the cancer is multiple myeloma.
14) A composition according to any of items 9-13, wherein the EZH2 inhibitor is EPZ-6438.
15) A composition according to any of items 9-14, wherein in the cytidine analogue is 5-Azacytidine.
16) A composition according to any of items 8-14, further comprising an Immunomodulatory drug (IMiD), preferably Lenalidomide and/or Pomalidomide.
17) An effective amount of an EZH2 inhibitor and a cytidine analogue for use in the preparation of a medicament for treating cancer.
18) A medicament according to item 17, wherein the cancer is resistant to Immunomodulatory drugs (IMiDs) chemotherapy.
19) A medicament according to any of items 17-18, wherein epigenetic changes in the cancer cells are restored.
20) A medicament according to any of items 17-19, wherein the epigenetic change is chromatin accessibility.
21) A medicament according to any of items 17-20, wherein the cancer is multiple myeloma.
22) A medicament according to any of items 17-21, wherein the EZH2 inhibitor is EPZ-6438.
23) A medicament according to any of items 17-22, wherein in the cytidine analogue is 5-Azacytidine.
24) A composition according to any of items 17-23, further comprising an Immunomodulatory drug (IMiD), preferably Lenalidomide and/or Pomalidomide.
25) A method for treating cancer comprising administering a combination of an EZH2 inhibitor and a cytidine analogue to a patient in need thereof.
26) A method for reversing drug resistance in a patient having an Immunomodulatory drug (IMiD) resistant cancer comprising administering to said patient a combination of an EZH2 inhibitor and a cytidine analogue.
27) A method for treating cancer comprising administering to a subject in need thereof
   (a) an effective amount of an EZH2 inhibitor, and
   (b) an effective amount of a cytidine analogue
   to provide a combination therapy having an enhanced therapeutic effect compared to the effect of the EZH2 inhibitor and the cytidine analogue administered alone.
28) A method according to item 27, wherein the combination therapy has a synergistic therapeutic effect.
29) A method according to any of items 25-28, further comprising administering at least one additional anti-cancer agent.
30) A method according to any of items 25-29, wherein said additional anti-cancer agent is an immunomodulatory drug (IMiD)
31) A method according to any of items 25-30, wherein the cancer is resistant to Immunomodulatory drugs (IMiDs) chemotherapy.
32) A method according to any of items 25-31, wherein epigenetic changes in the cancer cells is restored.
33) A method according to any of items 25-32, wherein the epigenetic change is chromatin accessibility.
34) A method according to any of items 25-33, wherein the cancer is multiple myeloma.
35) A method according to any of items 25-34, wherein the EZH2 inhibitor is EPZ-6438.
36) A method according to any of items 25-35, wherein in the cytidine analogue is 5-Azacytidine.
37) A kit for treating cancer comprising
   (a) an effective amount of an EZH2 inhibitor and
   (b) an effective amount of a cytidine analogue, and
   (c) optionally instruction for use thereof.

## Claims

1. A combination of an EZH2 inhibitor and a cytidine analogue for use in the treatment of cancer.

2. A combination according to claim 1, wherein the cancer is resistant to Immunomodulatory drugs (IMiDs) chemotherapy.

3. A combination according to any of claims 1-2, wherein the cancer is multiple myeloma.

4. A combination according to any of claims 1-3, wherein the EZH2 inhibitor is EPZ-6438.

5. A combination according to any of claims 1-4, wherein in the cytidine analogue is Azacytidine.

6. A combination according to any of claims 1-5, further comprising an Immunomodulatory drug (IMiD), preferably Lenalidomide and/or Pomalidomide.

7. A method for treating cancer comprising administering to a subject in need thereof
(a) an effective amount of an EZH2 inhibitor, and
(b) an effective amount of a cytidine analogue
to provide a combination therapy having an enhanced therapeutic effect compared to the effect of the EZH2 inhibitor and the cytidine analogue administered alone.

8. A method according to claim 7, wherein the combination therapy has a synergistic therapeutic effect.

9. A method according to any of claims 7-8, further comprising administering at least one additional anti-cancer agent.

10. A method according to any of claims 7-9, wherein said additional anti-cancer agent is an immunomodulatory drug (IMiD)

11. A method according to any of claims 7-10, wherein the cancer is resistant to Immunomodulatory drugs (IMiDs) chemotherapy.

12. A method according to any of claims 7-11, wherein epigenetic changes in the cancer cells is restored.

13. A method according to any of claims 7-12, wherein the epigenetic change is chromatin accessibility.

14. A method according to any of items 25-33, wherein the cancer is multiple myeloma.

15. A kit for treating cancer comprising
(a) an effective amount of an EZH2 inhibitor and
(b) an effective amount of a cytidine analogue, and
(c) optionally instruction for use thereof.
